(19) Europäisches Patentamt
European Patent Office
Office européen des brevets

(11) EP 4 479 741 B1

(12) FASCICULE DE BREVET EUROPEEN

(45) Date de publication et mention de la délivrance du brevet:
**01.04.2026 Bulletin 2026/14**

(21) Numéro de dépôt: **23709243.2**

(22) Date de dépôt: **13.02.2023**

(51) Classification Internationale des Brevets (IPC):
***G01N 33/26** (2006.01)* ***C10G 3/00** (2006.01)*
***C11B 3/00** (2006.01)* *C11B 3/04 (2006.01)*
*C11B 3/06 (2006.01)*

(52) Classification Coopérative des Brevets (CPC):
**G01N 33/26; C10G 3/50; C11B 3/006;** C11B 3/04; C11B 3/06

(86) Numéro de dépôt international:
**PCT/FR2023/050186**

(87) Numéro de publication internationale:
**WO 2023/156727 (24.08.2023 Gazette 2023/34)**

(54) **METHODE ET SYSTÈME DE DETERMINATION D'UN PARAMETRE D'UNE COMPOSITION D'ORIGINE NATURELLE PERMETTANT DE DETERMINER LE TRAITEMENT D'ELIMINATION EN HETEROATOMES LE PLUS APPROPRIE DE CETTE COMPOSITION**

VERFAHREN UND SYSTEM ZUR BESTIMMUNG EINES PARAMETERS EINER ZUSAMMENSETZUNG NATÜRLICHEN URSPRUNGS ZUR BESTIMMUNG DER GEEIGNETSTEN BEHANDLUNG ZUR ENTFERNUNG VON HETEROATOMEN AUS DIESER ZUSAMMENSETZUNG

METHOD AND SYSTEM FOR DETERMINING A PARAMETER OF A COMPOSITION OF NATURAL ORIGIN FOR DETERMINING THE MOST SUITABLE TREATMENT FOR THE REMOVAL OF HETEROATOMS FROM SAID COMPOSITION

(84) Etats contractants désignés:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorité: **15.02.2022 FR 2201327**

(43) Date de publication de la demande:
**25.12.2024 Bulletin 2024/52**

(73) Titulaire: **TotalEnergies OneTech**
**92400 Courbevoie (FR)**

(72) Inventeurs:
- **LIMOGES, Alice**
**69360 Solaize (FR)**
- **LAAGE, Ségolène**
**69360 Solaize (FR)**
- **PICARD, Florent**
**69360 Solaize (FR)**
- **LACOUX, Christine**
**69360 Solaize (FR)**

(74) Mandataire: **Fédit-Loriot**
**22, rue du Général Foy**
**75008 Paris (FR)**

(56) Documents cités:
- **GARCIA-MONTOTO VICTOR ET AL: "Phosphorus speciation analysis of fatty-acid-based feedstocks and fast pyrolysis biocrudes via gel permeation chromatography inductively coupled plasma high-resolution mass spectrometry", RSC ADVANCES, vol. 11, no. 43, 5 August 2021 (2021-08-05), pages 26732 - 26738, XP055959111, DOI: 10.1039/D1RA03470G**
- **VIIDANOJA JYRKI: "Analysis of phospholipids in bio-oils and fats by hydrophilic interaction liquid chromatography-tandem mass spectrometry", JOURNAL OF CHROMATOGRAPHY B, vol. 1001, 26 July 2015 (2015-07-26), NL, pages 140 - 149, XP055959130, ISSN: 1570-0232, DOI: 10.1016/j.jchromb.2015.07.036**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Processed by Luminess, 75001 PARIS (FR)

## Description

### Domaine de l'invention

La présente invention concerne une méthode de détermination d'un paramètre représentatif de la qualité d'une composition d'origine naturelle, ce paramètre permettant de déterminer le traitement d'élimination en hétéroatomes le plus approprié pour traiter cette composition, notamment en vue de son traitement pour la fabrication de carburants renouvelables, en particulier de type diesel et/ou carburant d'aviation (notamment les carburants d'aviation durable ou SAF : « Sustainable Aviation Fuel). L'invention concerne également un enchainement de procédés de traitement de ce type de composition, notamment pour fabriquer un carburant renouvelable.

### Art antérieur

Différents types de carburants renouvelables sont disponibles aujourd'hui : le biodiesel et le carburant issu de l'hydrotraitement d'huiles d'origine naturelle.

Le biodiesel est actuellement produit par transestérification de triglycérides avec du méthanol, produisant du méthyl-ester et du glycérol en présence d'un catalyseur basique homogène ou hétérogène. Les triglycérides proviennent d'huiles d'origine naturelle.

D'autres carburants renouvelables sont produits par hydrodésoxygénation d'huiles d'origine naturelle, généralement suivie d'une étape d'isomérisation permettant d'améliorer les propriétés à froid du carburant, et optionnellement d'une étape de fractionnement. Les étapes d'hydrodésoxygénation et d'isomérisation sont également réalisées en présence d'un catalyseur.

Les huiles d'origine naturelle habituellement utilisées pour fabriquer des carburants renouvelables, sont les graisses et huiles animales, diverses huiles à base de graines, soja, colza, palme, les huiles de graines qui ont été collectées après avoir été utilisées pour préparer des aliments, communément appelées huiles de cuisson usées (UCO), ou encore les huiles algales ou les huiles provenant des coques de fruits à coque, notamment les huiles de coques de noix de cajou.

Ces huiles et graisses peuvent cependant contenir des teneurs élevées en polluants (phospholipides, sels de phosphates, gommes, métaux, soufre, cendres, eau, pigments et autres matières indésirables) susceptibles d'avoir un effet délétère sur les traitements ultérieurs, avec par exemple désactivation des catalyseurs des procédés en aval, corrosion, encrassement, etc. Il est par conséquent nécessaire de les prétraiter afin d'éliminer tout ou partie de ces effets néfastes.

Ainsi, avant leur conversion en carburant renouvelable, les graisses et les huiles d'origine naturelle et les UCO sont habituellement prétraitées par des procédés chimiques et physiques bien connus, similaires à ceux mis en œuvre pour le traitement des huiles alimentaires, tels que dégommage (« degumming » en anglais), neutralisation avec une solution alcaline (généralement NaOH) ou acide (par exemple l'acide citrique) blanchiment (« bleaching » en anglais), finition ou polissage (« polishing » en anglais), traitement à la vapeur, etc.

Le document US2019338219A1 décrit ainsi un procédé de production de biodiesel comprenant une étape de prétraitement avant estérification. Le prétraitement est choisi en fonction de la teneur en acides gras de la charge. Il peut s'agir d'un raffinage chimique avec ajout d'un composé basique suivi d'une centrifugation puis d'un blanchiment et polissage, ou d'un dégommage avec ajout d'un composé basique également suivi d'une centrifugation puis d'un blanchiment et polissage, ou uniquement d'un blanchiment et polissage. L'étape de centrifugation permet d'éliminer la phase aqueuse, les composés polaires ou hydratables et les solides. L'étape de blanchiment (par chauffage ou ajout d'un agent de blanchiment) permet d'éliminer les solides, les savons résiduels, l'humidité et d'autres impuretés. L'étape de polissage permet d'éliminer par filtration les solides résiduels. S'il reste encore des acides gras, ils peuvent être éliminés soit par stripage (ou désacidification) ou convertis par estérification avant la réaction de trans-estérification. Le biodiesel produit est ensuite à nouveau soumis à une purification par filtration.

On connait aussi du document US2019031964A1 un procédé de purification permettant de réduire de manière notable le soufre, les métaux et autres impuretés dans des huiles renouvelables. L'huile est d'abord mélangée à une solution aqueuse d'acide citrique à 60°C, puis soumise à une centrifugation afin d'éliminer les gommes et enfin traitée avec un mélange contenant de l'eau, un composé ester à longue chaine hydrotraité et un dérivé phosphate à au moins 100°C pendant 10 minutes, puis la température est augmentée à 120°C-130°C pendant 30 à 90 minutes. Le mélange est ensuite refroidi et à nouveau centrifugé.

L'article Victor Garcia-Montoto et al.: "Phosphorus speciation analysis of fatty-acid-based feedstocks and fast pyrolysis biocrudes via gel permeation chromatography inductively coupled plasma high-resolution mass spectrometry", RSC ADVANCES, vol. 11, no. 43, 5 août 2021, pages 26732-26738, XP055959111, DOI: 10.1039/D1RA03470G, décrit une méthode de détermination de la qualité d'une composition d'origine naturelle des lipides dans le but de sélectionner ou ajuster un prétraitement. La méthode comprend une étape de détermination de la teneur totale de la composition en

phosphore et une étape d'identification des composés phosphorés.

Cependant, ces procédés bien connus consomment des produits chimiques, génèrent des déchets et peuvent consommer beaucoup d'énergie, en particulier lorsqu'il faut chauffer. En outre, en raison de la complexité des huiles, l'élimination de tous les produits de gomme peut être difficile, et ce d'autant plus que, la plupart du temps, des mélanges d'huiles et de graisses de différentes origines sont utilisés et leurs rapports relatifs peuvent fluctuer considérablement dans le temps. Il est donc nécessaire de disposer d'un procédé flexible et robuste pour éliminer correctement les phosphatides, les composés azotés et les métaux de mélanges complexes d'huiles et de graisses d'origines différentes et de composition fluctuante.

L'élimination des phosphatides, ainsi que des composés azotés, par les procédés de prétraitement habituels reste toutefois difficile et peu prédictible si bien que seuls les mélanges complexes d'huiles et de graisses répondant à des teneurs spécifiques en phosphore sont aujourd'hui acceptées à l'entrée de ces prétraitements, ce qui limite le choix des charges potentiellement utilisables. Ainsi, actuellement, les mélanges traités présentent une teneur totale en phosphore élémentaire inférieure ou égale à un seuil spécifique. Certaines de ces charges restent néanmoins difficiles à traiter même lorsqu'elles présentent une teneur en phosphore élémentaire inférieure à ce seuil.

Il existe donc un besoin pour traiter plus efficacement et à moindre coût les compositions d'origine naturelle comprenant des lipides et des hétéroatomes, notamment au moins un hétéroatome choisi parmi l'azote et le phosphore. Ces hétéroatomes sont typiquement sous la forme de composés organiques.

## Résumé

La demanderesse a découvert que, de manière surprenante, le rapport de la teneur totale en hétéroatome présent dans les composés hydratables à pH neutre sur la teneur totale en cet hétéroatome, notamment lorsque l'hétéroatome est le phosphore ou l'azote, permet de caractériser la qualité d'une composition d'origine naturelle contenant des lipides, et notamment son aptitude à être traitée efficacement par un traitement d'élimination des hétéroatomes.

L'objet de la présente invention est de proposer une méthode de détermination d'un paramètre représentatif de la qualité d'une composition d'origine naturelle contenant des lipides, permettant notamment de déterminer son aptitude à être traitée par un traitement d'élimination des hétéroatomes afin d'atteindre une teneur en hétéroatomes cible ou inférieure à une valeur cible de manière efficace et à moindre coût.

Ce paramètre peut être utilisé pour déterminer quel traitement d'élimination en hétéroatomes peut être appliqué à une composition d'huile d'origine naturelle contenant des lipides pour atteindre une teneur cible en hétéroatomes, notamment en phosphore et/ou azote.

Le paramètre peut aussi être utilisé dans un procédé de diminution de la teneur en hétéroatomes d'une composition d'origine naturelle contenant des lipides, notamment un procédé de diminution de la teneur en hétéroatomes

## Description détaillée

<u>Méthode de détermination d'un paramètre représentatif de la qualité d'une composition d'origine naturelle</u>

Un premier objet de l'invention concerne une méthode de détermination d'un paramètre représentatif de la qualité d'une composition d'origine naturelle comprenant des hétéroatomes et des lipides choisis parmi des lipides phénoliques, des acides gras, des triglycérides, des di-glycérides, des mono-glycérides, des phospholipides, des esters d'acides gras et/ou un mélange de deux ou plusieurs de ces composés, la méthode comprenant :

(A) la détermination de la teneur totale de ladite composition en au moins un hétéroatome choisi parmi le phosphore et l'azote,

(B) l'identification des composés de l'au moins un hétéroatome hydratables à pH neutre et la détermination de la teneur totale de ladite composition en l'au moins un hétéroatome présent dans les composés de cet hétéroatome hydratables à pH neutre identifiés,

(C) la détermination en tant que paramètre représentatif de la qualité de la composition du rapport de la teneur totale en l'au moins un hétéroatome présent dans les composés de cet hétéroatome hydratables à pH neutre identifiés sur la teneur totale de la composition en cet hétéroatome, ce rapport étant déterminé pour le phosphore, pour l'azote ou pour chacun de ces deux hétéroatomes.

En particulier, l'étape (B) peut être réalisée au moyen d'une technique d'analyse choisie parmi la RMN, la spectroscopie de masse, la chromatographie ionique, la chromatographie en phase liquide couplée à la spectrométrie de

masse (LC-MS), la chromatographie sur couche mince performante (HPTLC).

On pourra ainsi utiliser la RMN du phosphore 31 (noté $^{31}P$) ou de l'azote 15 (noté $^{15}N$).

La détermination réalisée à l'étape (A) peut être réalisée par une analyse élémentaire, typiquement par fluorescence X (XRF) ou encore par ICP (notamment selon la norme UOP 389), bien que d'autres méthodes soient envisageables (RMN, HPTLC,...).

Les teneurs totales peuvent être des teneurs en masse, par exemple exprimée en ppm, ou des concentrations, par exemple une concentration molaire ou autre.

Les techniques d'analyse susmentionnées sont bien connues de l'homme du métier qui saura les mettre en œuvre, et notamment préparer les échantillons, pour identifier les composés hydratables à pH neutre et quantifier le phosphore et/ou l'azote présents dans cette composition.

Par exemple, pour la RMN du $^{31}P$, l'échantillon peut subir un lavage avec un agent chélatant (par exemple de type EDTA ou autre) à un pH approprié (par exemple un pH=7) puis les phases organique et aqueuse sont séparées et l'analyse par RMN du $^{31}P$ est ensuite réalisée sur chacune des phases récupérées.

Typiquement, les composés de l'azote ou du phosphore hydratables à pH neutre que l'on souhaite identifier et dont on souhaite quantifier la teneur en P et/ou N sont des composés dont on connaît la structure et la présence potentielle, par exemple suite à une analyse préalable de la composition.

<u>Composition d'origine naturelle</u>

La composition d'origine naturelle utilisée dans la présente invention est une composition comprenant des hétéroatomes et des lipides choisis parmi des lipides phénoliques, des acides gras, des triglycérides, des di-glycérides, des mono-glycérides, des phospholipides, des esters d'acides gras et/ou un mélange de deux ou plusieurs de ces composés.

Cette composition peut comprendre, ou consister en, une huile d'origine naturelle ou un mélange d'huiles naturelles.

Une huile d'origine naturelle est définie comme une huile qui ne contient pas d'huile minérale d'origine fossile.

La composition selon l'invention peut contenir une ou plusieurs huiles d'origine naturelle choisie parmi une huile végétale, une huile ou une graisse animale, une huile usagée, une huile produite par des microorganismes, des esters résultant de la trans-estérification des esters d'acides gras contenus dans une ou plusieurs de ces huiles, ainsi que leurs mélanges.

Typiquement, une huile d'origine naturelle peut contenir 50%m ou plus, 60%m ou plus, préférentiellement 70%m ou plus, de lipides phénoliques, d'acides gras et/ou d'esters d'acides gras (mono-, di-, triglycérides, esters éthyliques d'acides gras, esters méthyliques d'acides gras).

Une huile d'origine naturelle peut contenir 50%m ou plus d'esters d'acides gras (mono-, di-, triglycérides, esters éthyliques d'acides gras, esters méthyliques d'acides gras) et/ou d'acides gras, de préférence 60%m ou plus, préférentiellement 70%m ou plus.

Dans un mode de réalisation, une huile d'origine naturelle ou un mélange d'huiles d'origine naturelle, peut contenir des esters d'acides gras et des acides gras libres, contenant un à trois groupes acyle en C8-C24, saturés ou insaturés. Lorsque plusieurs groupes acyles sont présents, ils peuvent être identiques ou différents.

Des compositions résultant de la trans-estérification des esters d'acides gras contenus dans ces huiles, telles que des compositions comprenant des esters méthyliques d'acides gras ou des esters éthyliques d'acides gras, et comportant des impuretés provenant des huiles, peuvent également faire partie des compositions d'origine naturelle traitées considérées dans la présente invention.

Une huile d'origine naturelle peut contenir 50%m ou plus, de préférence 60%m ou plus, préférentiellement 70% m ou plus, de lipides phénoliques. Ces lipides phénoliques comprennent notamment les composés représentés par la formule (1):

[Chem 1]

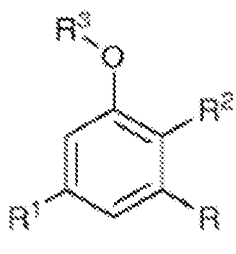

(1)

Où :

R est un groupement alkyle à chaine linéaire en C10-C30, optionnellement en C12-C20, par exemple en C15, saturé ou non, substitué ou non par des hétéroatomes choisis parmi O, N ou S,

$R^1$ est l'hydrogène ou un groupe hydroxyle,

$R^2$ est l'hydrogène, un groupe carboxylique ou un ester,

$R^3$ est l'hydrogène.

L'huile d'origine naturelle peut notamment comprendre un ou plusieurs des lipides phénoliques suivants :

- les phénols alkylés dont le groupement alkyle est à chaine linéaire en C10-C30, optionnellement en C12-C20, par exemple en C15, saturé ou non, substitué ou non par des hétéroatomes choisis parmi O, N ou S,
- les alkylrésorcinols dont le groupement alkyle est à chaine linéaire en C10-C30, optionnellement en C12-C20, par exemple en C15, saturé ou non, substitué ou non par des hétéroatomes choisis parmi O, N ou S,
- les acides anacardiques, dont le groupement alkyle est à chaine linéaire en C10-C30, optionnellement en C12-C20, par exemple en C15, saturé ou non, substitué ou non par des hétéroatomes choisis parmi O, N ou S.

L'huile végétale peut être choisie parmi l'huile de pin, l'huile de colza, l'huile de tournesol, l'huile de ricin, l'huile d'arachide, l'huile de lin, l'huile de babasu, l'huile de chanvre, l'huile de linola, l'huile de jatropha, l'huile d'arachide, l'huile de son de riz, l'huile de moutarde, l'huile de carinata l'huile de noix de coco, l'huile de coprah, l'huile d'olive, l'huile de palme, l'huile de coton, l'huile de maïs, l'huile de palmiste, l'huile de soja, l'huile de courge, l'huile de pépin de raisin, l'huile d'argan, l'huile de jojoba, l'huile de sésame, l'huile de noix, l'huile de noisette, l'huile de bois de Chine, l'huile de riz, l'huile de carthame, l'huile d'algues, les huiles usagées, l'huile de coque de fruit à coque (notamment l'huile de coque de noix de cajou), et toute combinaison de celles-ci.

L'huile usagée comprend les huiles de cuisson usagées (huiles alimentaires usagées) et les huiles récupérées à partir des eaux résiduelles, telles que les graisses/huiles de piège et de vidange, les huiles de gouttière, les huiles d'égout, par exemple des stations d'épuration des eaux, et les graisses usagées de l'industrie alimentaire.

La graisse animale peut être choisie parmi le suif, le saindoux, la graisse (graisse jaune et brune), l'huile/graisse de poisson, la matière grasse du lait.

L'huile d'origine naturelle peut aussi être une huile produite par des micro-organismes, naturels ou génétiquement modifiés, tels que des bactéries, des levures, notamment des levures oléagineuses, des algues, des procaryotes ou des eucaryotes. En particulier, ces huiles peuvent être récupérées par des méthodes d'extraction mécanique ou chimique bien connues.

Les huiles d'origine naturelle susmentionnées, dont la plupart sont riches en triglycérides ou en lipides phénoliques, contiennent en outre des quantités variables de composants tels que des acides gras libres, des mono et di-glycérides, et/ou de nombreux autres composants organiques et inorganiques, notamment des phosphatides, des stérols, des tocophérols, des tocotriénols, des hydrocarbures, des pigments (gossypol, chlorophylle), des vitamines (caroténoïdes), des stérols glucosides, des glycolipides, des fragments de protéines, des traces de pesticides et des traces de métaux, ainsi que des matières résineuses et mucilagineuses. Parmi ces derniers composants, certains composés, notamment ceux qui contiennent des hétéroatomes, sont des polluants qu'il est préférable d'éliminer au moins en partie avant un traitement ultérieur.

La composition d'huile renouvelable selon l'invention comprend ainsi typiquement des hétéroatomes dont notamment le phosphore et/ou l'azote. Ces hétéroatomes sont généralement sous forme de composés organiques, notamment sous forme de lipides.

La teneur en phosphore de la composition d'origine naturelle peut être de 20ppm ou plus ou de 50ppm ou plus, par exemple de 50ppm à 1500 ppm, ou de 200ppm à 1200ppm, mesurée par exemple par fluorescence X ou ICP par la méthode UOP 389.

La teneur en azote de la composition d'origine naturelle peut être de 50ppm ou plus, par exemple de 50ppm à 1200ppm ou de 200ppm à 2000 ppm, mesurée par exemple par fluorescence X.

La composition d'origine naturelle peut en outre comprendre un ou plusieurs autres hétéroatomes tels que des métaux alcalins, notamment le potassium, des métaux alcalino-terreux, et/ou du chlore. La teneur en ces hétéroatomes peut être variable selon les constituants de la composition. Elle peut être déterminée par une analyse élémentaire de type fluorescence X ou par ICP.

Dans la composition d'origine naturelle considérée dans l'invention, le phosphore peut notamment être présent sous la forme de phosphatides, dont les plus courants sont l'acide phosphatique, la phosphatidyléthanolamine, la

phosphatidylcholine, le phosphatidylinositol et les sels de phosphates. Comme ces composés sont souvent chargés en raison de leur pKa faible (groupe phosphate) ou élevé (groupe amino), ils peuvent également contenir des éléments alcalins ou alcalino-terreux ou absorber des cations métalliques comme le cuivre ou le fer.

La figure 3 représente les différentes formes de ces phospholipides dans l'eau en fonction du pH, le groupement R représente un di-glycéride. On notera ainsi que la phosphatidylcholine (notée PC) et le phosphatidylinositol (noté PI) sont hydratables quel que soit le pH, que la phosphatidyléthanolamine (notée PE) est hydratable à pH 2-3, alors que l'acide phosphatique (noté PA) est hydratable pour des pH supérieurs à 4 s'il n'est pas chélaté par des cations présents dans le mélange, auquel cas il restera dans la phase organique.

Par « composé hydratable », on entend un composé au moins partiellement soluble dans l'eau. Cette solubilité peut dépendre du pH, comme expliqué ci-dessus.

Dans la présente invention, on considèrera en tant que composés hydratables à pH neutre (pH = 7), les composés au moins partiellement solubles ou entièrement solubles dans l'eau à pH neutre, notamment dans les conditions de température du traitement mis en œuvre. On considère qu'un composé est au moins partiellement soluble dans l'eau à pH neutre quand au moins 40%m de ce composé est solubilisé, notamment dans les conditions de température du traitement mis en œuvre. Les conditions de température du traitement mis en œuvre sont notamment celles du lavage à l'eau à pH neutre décrit plus loin.

Les composés du phosphore hydratables à pH neutre sont notamment les sels hydratables de phosphate, la phosphatidylcholine et le phosphatidylinositol, et, dans une moindre mesure, le phosphatidylglycérol.

L'azote est présent dans la phosphatidylcholine et la phosphatidyléthanolamine. Il peut également être présent dans la composition d'origine naturelle sous la forme de chlorophylle, résidus de protéines, amines grasses, ...

Les composés de l'azote hydratables à pH neutre sont notamment la phosphatidylcholine et, dans une moindre mesure, le phosphatidylinositol.

L'invention n'est toutefois pas limitée à ces composés, et on pourra notamment prendre en compte tous les composés hydratables à pH neutre identifiables.

Notamment, afin d'identifier et de quantifier les composés hydratables à pH neutre, on pourra procéder à une analyse de la composition à traiter de manière à identifier les composés organiques contenant du phosphore et/ou de l'azote, isoler ces composés et procéder à des tests de solubilité dans l'eau, notamment à différents pH dans les conditions de température du traitement mis en œuvre. L'identification des composés pourra notamment être réalisée au moyen des techniques précédemment citées (RMN, la spectroscopie de masse, la chromatographie ionique, la chromatographie en phase liquide couplée à la spectrométrie de masse et la chromatographie sur couche mince performante).

<u>Procédé de détermination du traitement d'élimination</u>

Un autre objet de l'invention concerne un procédé de détermination du traitement d'élimination en hétéroatomes à appliquer à une composition d'huile d'origine naturelle, comprenant des hétéroatomes et des lipides choisis parmi des lipides phénoliques, des acides gras, des triglycérides, des di-glycérides, des mono-glycérides, des phospholipides, des esters d'acides gras et/ou un mélange de deux ou plusieurs de ces composés, pour atteindre une teneur cible en au moins un hétéroatome, notamment le phosphore et/ou l'azote.

Le procédé selon l'invention comprend :

(a) une étape de détermination d'au moins un paramètre représentatif de la qualité de la composition au moyen de la méthode de détermination selon l'invention,

(b) une étape d'estimation d'une teneur résiduelle en au moins un hétéroatome choisi parmi le phosphore et l'azote, à partir du paramètre représentatif, cette teneur résiduelle correspondant à la teneur en l'au moins un hétéroatome après élimination des composés hydratables à pH=7 contenant cet hétéroatome,

(c) une étape de détermination d'un traitement à appliquer à ladite composition pour atteindre une teneur cible en au moins un hétéroatome, notamment choisi parmi le phosphore et/ou l'azote, en sortie du traitement, ledit traitement étant choisi parmi

(i) un lavage avec de l'eau à pH neutre,

(ii) un lavage avec de l'eau à pH neutre suivi d'une étape d'élimination de composés non hydratables,

(iii) un lavage avec de l'eau à pH neutre suivi d'une étape d'élimination de composés hydratables à un pH autre qu'un pH neutre,

(iv) un lavage avec de l'eau à pH neutre suivi d'une étape d'élimination de composés hydratables à un pH autre qu'un pH neutre puis d'une étape d'élimination de composés non hydratables,

dans laquelle

on compare la dite teneur résiduelle à la teneur cible et

on choisit le traitement (i) si la teneur résiduelle est inférieure ou égale à la teneur cible,

sinon, on utilise une base de données ou un modèle configuré pour fournir, une estimation d'une teneur en l'au moins un hétéroatome en sortie de chaque traitement (ii) à (iv) en fonction d'une teneur en cet hétéroatome en entrée du traitement égale à la teneur résiduelle, et on choisit le traitement pour lequel la teneur en sortie estimée est inférieure ou égale à la teneur cible, et optionnellement pour lequel le nombre d'étapes de traitement est le plus faible.

Il est ainsi possible de déterminer le traitement, notamment le traitement présentant le moins d'étapes, permettant d'obtenir une teneur cible en hétéroatomes, notamment en P et/ou N. Le traitement permet ainsi d'atteindre cette teneur cible quelle que soit la teneur initiale en phosphore et/ou azote de la composition.

On pourra notamment choisir un traitement permettant d'atteindre une teneur cible soit pour le phosphore, soit pour l'azote, soit pour les deux. On déterminera alors à l'étape (a) le paramètre représentatif de la qualité de la composition pour le phosphore, le paramètre représentatif de la qualité de la composition pour l'azote, ou les deux paramètres, et la base de données ou le modèle utilisés à l'étape (c) sont alors configurés pour fournir une estimation d'une teneur en phosphore, d'une teneur en en azote, ou les deux, en sortie de chaque traitement (ii) à (iv) en fonction d'une teneur en cet hétéroatome en entrée du traitement égale à la teneur résiduelle.

Il peut arriver que, au cours de l'étape (c), aucune des teneurs en sortie estimées ne soit inférieure ou égale à la teneur cible. Dans ce cas, on peut avantageusement réitérer les étapes (a) à (c) en diluant ladite composition d'origine naturelle avec une autre composition d'origine naturelle, notamment une composition présentant un paramètre représentatif de sa qualité supérieur à celui de ladite composition (par exemple préalablement déterminé par la méthode de détermination selon l'invention).

Ceci peut permettre d'utiliser une charge pour laquelle les traitements d'élimination en hétéroatomes usuels ne sont pas suffisamment efficaces pour réduire suffisamment la quantité en hétéroatomes, notamment en phosphore et/ou azote, pour atteindre une spécification cible.

Avantageusement, la base de données et/ou le modèle peut être configuré pour fournir une estimation des teneurs l'au moins un hétéroatome en sortie dudit traitement obtenues pour différentes conditions opératoires de chaque traitement (i) à (iv) et au cours de l'étape (c), on peut alors choisir un traitement et les conditions opératoires de ce traitement pour lequel la teneur en sortie estimée est inférieure ou égale à la teneur cible, et optionnellement pour lequel le nombre d'étapes de traitement est le plus faible et/ou pour lequel la quantité de réactif utilisée est la plus faible. Il est ainsi possible d'optimiser les conditions de traitement pour chaque charge à traiter, ce qui permet notamment de réduire les quantités de réactifs utilisées, et donc les coûts.

Le procédé de détermination selon l'invention peut notamment être utilisé pour une composition d'origine naturelle telle que définie ci-dessus.

La base de données ou le modèle utilisés dans le présent procédé sont ainsi configurés pour fournir des estimations de teneurs en hétéroatomes (au moins en P et/ou N) en sortie d'un traitement. Ils peuvent être préalablement établis au moyen de tests au cours desquels des compositions d'origine naturelle sont soumises à chacun des traitements (i) à (iv). On pourra ainsi mesurer pour chaque test et chaque type de traitement, optionnellement pour différentes conditions opératoires de chaque traitement :

- la teneur totale de chaque composition en au moins un hétéroatome choisi parmi le phosphore et l'azote, avant et après traitement,
- la teneur totale de chaque composition en l'au moins un hétéroatome contenu dans les composés de cet hétéroatome hydratables à pH neutre.

Le procédé de détermination selon l'invention peut en outre comprendre une étape (d) de traitement de ladite composition, dans laquelle on soumet celle-ci au traitement choisi à l'étape (c) et l'on récupère une composition d'origine naturelle présentant une teneur réduite en au moins un hétéroatome, notamment en au moins un hétéroatome choisi parmi le phosphore et l'azote, et avantageusement en au moins un autre hétéroatome, notamment choisi parmi les métaux, les métaux alcalins, les alcalino-terreux et le chlore.

La composition ainsi traitée peut ensuite être soumise à une étape d'hydrotraitement, optionnellement en

mélange avec des hydrocarbures d'origine fossile, puis avantageusement à une étape d'isomérisation, afin de produire un carburant renouvelable, notamment de type diesel.

Alternativement, la composition ainsi traitée peut être soumise à une étape de trans-estérification ou d'estérification pour produire un carburant de type biodiesel.

Les étapes des traitements d'élimination (i) à (iv) ainsi que l'étape d'hydrotraitement peuvent être tel que décrit plus bas en référence au procédé de diminution de la teneur en hétéroatomes d'une composition d'origine naturelle selon l'invention.

Procédé de diminution de la teneur en hétéroatomes

L'invention a également pour objet un procédé de diminution de la teneur en hétéroatomes d'une composition d'origine naturelle comprenant des hétéroatomes et des lipides choisis parmi des lipides phénoliques, des acides gras, des triglycérides, des di-glycérides, des mono-glycérides, des phospholipides, des esters d'acides gras et/ou un mélange de deux ou plusieurs de ces composés, notamment telle que définie plus haut.

Dans un premier mode de réalisation, le procédé comprend :

(E1) une étape de détermination du traitement d'élimination en hétéroatomes à appliquer à la composition d'origine naturelle pour atteindre une teneur cible en au moins un hétéroatome, au moyen du procédé de détermination selon l'invention, et

(E2) une étape au cours de laquelle on soumet ladite composition d'origine naturelle au traitement déterminé à l'étape (E1).

Dans un deuxième mode de réalisation, le procédé comprend :

(E'2) une étape d'élimination en hétéroatomes au cours de laquelle on soumet la composition d'origine naturelle à un traitement choisi parmi :

(i) un lavage avec de l'eau à pH neutre,

(ii) un lavage avec de l'eau à pH neutre suivi d'une étape d'élimination de composés non hydratables,

(iii) un lavage avec de l'eau à pH neutre suivi d'une étape d'élimination de composés hydratables à un pH autre qu'un pH neutre,

(iv) un lavage avec de l'eau à pH neutre suivi d'une étape d'élimination de composés hydratables à un pH autre qu'un pH neutre puis d'une étape d'élimination de composés non hydratables,

et dans lequel, la composition d'origine naturelle présente un paramètre représentatif de la qualité de la composition, préalablement déterminé au moyen de la méthode de détermination selon l'invention, qui est supérieur ou égal à un seuil au-delà duquel l'efficacité de l'étape d'élimination est suffisante pour réduire la teneur en hétéroatomes de ladite composition à une teneur cible ou inférieure à une teneur cible.

Ceci permet de sélectionner, en amont de l'étape d'élimination, une composition d'origine naturelle pour laquelle un traitement particulier permet d'obtenir une spécification particulière. Ceci peut notamment permettre d'optimiser le fonctionnement d'une unité de traitement existante.

Ces valeurs seuils du paramètre peuvent être déterminées préalablement à partir d'une base de données identique à celle précédemment décrite, par exemple par un traitement statistique des données de la base et/ou au moyen d'un modèle tel que précédemment décrit.

Chacun des quatre traitements (i) à (iv) envisageable comprend une étape de lavage avec de l'eau à pH neutre, qui peut être suivie, selon le traitement, d'une étape d'élimination de composés non hydratables et/ou d'une étape d'élimination de composés hydratables à un pH autre qu'un pH neutre. Chacune de ces étapes est détaillée ci-après.

Dans chacun des modes de réalisation, le procédé de diminution peut en outre comprendre une étape d'hydrotraitement (E3) du produit directement issu de l'étape (E2) ou (E'2).

Etape de lavage avec de l'eau à pH neutre

Un lavage à l'eau à pH neutre de ladite composition d'origine naturelle permet de diminuer de manière notable la

teneur en hétéroatomes, notamment en hétéroatomes choisis parmi le phosphore, l'azote, le chlore, les métaux alcalins, les alcalino-terreux, en particulier elle permet d'éliminer les composés de l'azote et/ou du phosphore contenus dans des composés de ces atomes hydratables à pH neutre.

Cette étape de lavage à l'eau est réalisée avec une eau à pH neutre, autrement dit sans ajout de produit chimique (acide ou alcalin) visant à modifier le pH. Il s'agit typiquement d'un lavage avec une eau déminéralisée. Ce lavage est généralement réalisé avec une agitation pour favoriser le contact entre l'eau et l huile.

Lorsque la teneur résiduelle en hétéroatomes (notamment P et/ou N) estimée à partir du paramètre représentatif est inférieure à la teneur cible, ce lavage peut être suffisant pour éliminer la totalité, ou la quasi-totalité, de ces hétéroatomes sans avoir à réaliser d'autres traitements d'élimination des hétéroatomes tels que les étapes d'éliminations supplémentaires prévues dans les traitements (ii) à (iv).

Typiquement, ce lavage à l'eau est réalisé à pression atmosphérique, à une température allant de la température ambiante à une température à laquelle la composition est liquide. Cette température pourra être déterminée en fonction du point d'écoulement de la composition. Elle peut être supérieure à 50°C, par exemple de 50°C à 100°C.

Cette étape de lavage est typiquement réalisée par mise en contact de la composition avec de l'eau à pH neutre suivie d'une séparation des phases huileuse et aqueuse, la phase huileuse récupérée formant une composition présentant une teneur réduite en composés hydratables à pH neutre. Cette mise en contact peut être réitérée une ou plusieurs fois. Après chaque mise en contact, la phase huileuse et la phase aqueuse sont alors séparées, et la phase huileuse séparée est mise en contact à nouveau avec de l'eau à pH neutre ou récupérée. Cette séparation peut être réalisée au moyen de toute technique de séparation usuelle.

On pourra notamment réaliser ce lavage par mise en contact de la composition avec de l'eau à pH neutre, de préférence sous agitation, pendant une durée suffisante pour mélanger les deux phases, par exemple pendant une durée de 15min à 1h.

Avantageusement, lors de l'étape de lavage, le ratio en masse eau /composition peut être de 1 : 99 à 10 : 90. De faibles quantités d'eau suffisent en effet à abaisser notablement la teneur en hétéroatomes.

Cette étape de lavage peut être réalisée dans une enceinte spécifique, dédiée à cette étape. On pourra par exemple utiliser une enceinte disposant d'un dispositif de mélange eau/huile (pompe de recirculation avec injection d'eau en amont de la pompe, dispositif d'agitation, ...).

Toutefois, avantageusement, l'étape de lavage peut aussi être mise en œuvre dans une enceinte choisie parmi un bac de stockage, une capacité de dessalage, un ballon, notamment un ballon séparateur de type eau/huile. Le lavage peut avantageusement être effectué dans une enceinte déjà existante d'une unité de traitement.

Le produit issu de l'étape de lavage est récupéré de manière usuelle par une technique de séparation eau/huile, par exemple par centrifugation et/ou décantation et/ou avec application d'un champ électrique. Lorsque la séparation est réalisée par décantation, elle peut être réalisée dans la même enceinte que celle ayant servi au lavage.

Le produit récupéré, notamment directement, à l'issue de cette étape de lavage peut soit constituer une composition raffinée, notamment une huile raffinée qui peut être soumise ensuite directement à une étape d'hydrotraitement, soit une huile partiellement raffinée, qui peut être soumise ensuite directement soit à une étape d'élimination des composés hydratables à un pH autre qu'un pH neutre puis à une étape d'élimination des composés non hydratables, soit uniquement à une étape d'élimination des composés non hydratables, avant une étape ultérieure telle qu'un hydrotraitement.

Etape d'élimination de composés hydratables à un pH autre qu'un pH neutre (à pH basique ou pH acide)

Typiquement, cette étape permet d'éliminer les composés hydratables, notamment du phosphore, de l'azote et des métaux, et une partie du chlore, qui n'ont pas été éliminés lors de l'étape de lavage à l'eau à pH neutre.

Cette étape d'élimination peut comprendre un ou plusieurs traitements choisis par exemple parmi un traitement de dégommage en milieu acide ou basique et un traitement par cavitation, chaque traitement étant suivi d'une étape de séparation des phases huileuse et aqueuse, la phase huileuse récupérée formant une composition présentant une teneur réduite en composés hydratables à un pH autre qu'un pH neutre. Cette séparation peut être réalisée au moyen de toute technique de séparation usuelle.

On pourra notamment réaliser plusieurs traitements de dégommage dans des milieux acides et basiques afin d'éliminer les composés hydratables à pH acide et basique respectivement.

Le traitement de dégommage peut être un traitement de dégommage à l'eau au cours duquel l'huile à traiter est typiquement chauffée à 60-70 °C, de l'eau additivée en composé basique (par exemple NaOH) ou acide (par exemple acide citrique ou phosphorique) est ajoutée et mélangée pendant environ 30 minutes, puis les gommes hydratées sont séparées par centrifugation et l'huile dégommée est séchée sous vide. Ce procédé implique souvent l'ajout de vapeur vive au produit à traiter pendant une courte période. La quantité d'eau appropriée représente normalement environ 75 % en poids de la teneur en phosphatides de l'huile à traiter. Une quantité insuffisante d'eau produit des gommes visqueuses sombres, tandis qu'une quantité excessive d'eau entraîne des pertes excessives de l'huile par hydrolyse. Une huile

dégommée à l'eau contient généralement encore des phosphatides (entre 50 et 200 ppm en masse).

Le traitement de dégommage peut être un traitement à l'acide au cours duquel l'huile à traiter est typiquement chauffée à 60-70°C, et un mélange eau-acide est ajouté et mélangé pendant environ 30 minutes. On utilise typiquement l'acide phosphorique ou l'acide citrique.

Le traitement de dégommage peut être un traitement de dégommage enzymatique dans lequel une enzyme, par exemple la phospholipase A1, l'enzyme de dégommage la plus récente, transforme les phospholipides en lysophospholipides et en acides gras libres. Ce processus comporte trois étapes importantes :

(1) ajustement du pH avec un tampon ;

(2) réaction enzymatique dans les bassins de rétention ; et

(3) la séparation de la boue et de l'huile.

L'huile à dégommer par voie enzymatique de cette manière peut être brute ou préalablement dégommée à l'eau.

Le manuel des lipides (The lipid handbook, édité par Frank D. Gunstone, John L. Harwood, Albert J. Dijkstra. 3rd ed.) décrit de nombreuses variantes et détails des traitements de dégommage.

Le traitement peut être un traitement par cavitation, et notamment par cavitation hydrodynamique, de l'huile à traiter en présence d'eau dans des conditions efficaces pour générer des caractéristiques de cavitation et pour transférer au moins une partie des impuretés contenues dans l'huile dans une phase aqueuse.

La cavitation est le phénomène de formation de bulles de vapeur dans un liquide en écoulement dans les régions où la pression du liquide est inférieure à sa pression de vapeur à la température considérée.

La cavitation est un phénomène de nucléation, de croissance et d'implosion (effondrement) de cavités remplies de vapeur ou de gaz, qui peut être obtenu par le passage d'ultrasons (cavitation acoustique), par un laser, par l'injection de vapeur dans un fluide froid ou par des modifications de l'écoulement et de la pression (cavitation hydrodynamique).

La cavitation hydrodynamique peut être générée en faisant passer le mélange à traiter à travers un ou plusieurs dispositifs de cavitation.

Le procédé de cavitation hydrodynamique peut donc comprendre les étapes suivantes :

le pompage de l'huile à traiter à travers un dispositif de cavitation,

la génération de caractéristiques de cavitation pour éliminer les impuretés.

Des dispositifs de cavitation appropriés qui peuvent être utilisés sont par exemple divulgués dans WO201098783A1, US8911808B2, US7762715B2, US8042989B2.

Par exemple, un dispositif de cavitation approprié comprend un chemin d'écoulement à travers lequel le fluide est pompé, tel que celui divulgué dans US8911808B2, dans lequel une pression de pompe prédéterminée est appliquée de préférence dans la plage de 340 kPa-34 MPA.

Dans le traitement par cavitation hydrodynamique, les phosphatides sont hydratés en gommes, qui sont insolubles dans l'huile et peuvent être facilement séparés sous forme de boue formant une phase aqueuse, par exemple par décantation, filtrage ou action centrifuge.

Dans un mode de réalisation, le traitement par cavitation, notamment par cavitation hydrodynamique peut être réalisée en présence d'un agent de dégommage. Ce dernier peut être choisi parmi l'eau, la vapeur, les acides, les agents complexants et leurs mélanges.

Les acides sont par exemple des acides forts, en particulier des acides inorganiques, tels que l'acide phosphorique, l'acide sulfurique.

Les agents complexants sont par exemple des acides organiques faibles (ou leurs anhydrides correspondants) tels que l'acide acétique, l'acide citrique, l'acide oxalique, l'acide tartrique, l'acide malique, l'acide maléique, l'acide fumarique, l'acide aminé aspartique, l'acide éthylènediaminetétraacétique (EDTA).

De préférence, l'agent de dégommage comprend de l'eau, de la vapeur, de l'acide phosphorique, de l'acide acétique, de l'acide citrique, de l'acide oxalique, de l'acide tartrique, de l'acide malique, de l'acide fumarique, de l'acide aminé aspartique, de l'acide éthylènediaminetétraacétique, une base, des sels, des agents chélateurs, des éthers couronnes ou de l'anhydride maléique.

Le traitement par cavitation peut être réalisé à des températures proches de la température ambiante ou inférieures à celle-ci, par exemple à 15-25°C. Toutefois, la cavitation hydrodynamique peut être réalisée entre 10 et 90°C, de préférence entre 25 et 75°C et plus préférablement entre 30 et 60°C.

On pourra par exemple procéder tel que décrit dans le document WO2019229035A1.

Le produit issu de cette étape d'élimination peut être séparé par une ou plusieurs des techniques connues

suivantes : sédimentation, centrifugation, filtration, distillation, extraction ou lavage, de préférence sédimentation, centrifugation, filtration. Le produit récupéré après séparation de l'eau aqueuse est une phase huileuse qui constitue une composition ou huile partiellement raffinée qui peut ensuite être soumise à une étape d'élimination de composés non hydratables.

Etape d'élimination de composés non hydratables

Typiquement, cette étape permet d'éliminer les composés non hydratables, notamment du phosphore, de l'azote, tels que les phosphatides non hydratables (sels de calcium et de magnésium de l'acide phosphatique et de la phosphatidyl éthanolamine), ainsi que d'autres éléments tels que le soufre, et/ou une partie du chlore organique résiduel, qui n'ont pas été éliminés lors de l'étape de lavage à l'eau à pH neutre et l'étape optionnelle d'élimination des composés hydratables à un pH autre qu'un pH neutre.

Cette étape comprend typiquement un ou plusieurs traitements choisis parmi un traitement de blanchiment dans lequel le produit à traiter est mis en contact avec un absorbant, un traitement dans lequel le produit à traiter est mis en contact avec une résine échangeuse d'ions, un lavage acide doux, un traitement utilisant des lits de garde, une filtration, une extraction par solvant, chaque traitement étant suivi d'une étape de séparation des phases huileuse et aqueuse, la phase huileuse récupérée formant une composition présentant une teneur réduite en composés non hydratables. Cette séparation peut être réalisée au moyen de toute technique de séparation.

Le blanchiment est une technique bien connue, généralement utilisée pour décolorer et purifier les huiles raffinées chimiquement ou physiquement. Elle assure généralement l'élimination des savons, des phosphatides résiduels, des métaux à l'état de traces et de certains produits d'oxydation. Elle catalyse l'élimination du carotène et l'adsorbant catalyse également la décomposition des peroxydes. Une autre fonction est l'élimination des peroxydes et des produits d'oxydation secondaires.

Ce traitement consiste à mettre en contact le produit à traiter avec un absorbant, tel que les argiles adsorbantes, la silice amorphe synthétique et les charbons actifs. Optionnellement, préalablement à cette mise en contact, le produit à traiter peut être mélangé avec un acide afin de décomposer les complexes ion métallique/phosphatides.

Les paramètres clés du traitement de blanchiment sont le type et le dosage de l'adsorbant, la température, le temps, l'humidité et la filtration, comme indiqué dans le Lipids handbook, édité par Frank D. Gunstone, John L. Harwood, Albert J. Dijkstra. 3rd ed., chapitre 3.7) ; ou encore dans le « practical guide to vegetable oil processing", 2nd edition, Monoj. K. Gupta.

Un autre traitement possible est un traitement par résine échangeuse d'ions. Ce traitement consiste à mettre en contact le produit à traiter avec une résine échangeuse d'ions dans une zone de prétraitement, dans des conditions de prétraitement. La résine échangeuse d'ions est par exemple une résine échangeuse d'ions acide telle que l'AmberlystTM-15 et peut être utilisée comme lit dans un réacteur à travers lequel le produit à traiter s'écoule, soit en amont, soit en aval.

Un autre traitement possible est un lavage à l'acide doux. Ce traitement est effectué en mettant en contact le produit à traiter avec un acide tel que l'acide sulfurique, nitrique, phosphorique ou chlorhydrique dans un réacteur. L'acide et le produit à traiter peuvent être mis en contact dans un processus discontinu ou continu. La mise en contact se fait avec une solution acide diluée, généralement à température ambiante et à la pression atmosphérique. Si la mise en contact est effectuée de manière continue, elle se fait généralement à contre-courant.

Un autre traitement possible est l'utilisation de lits de garde, bien connus dans l'art. Il peut s'agir de lits de garde contenant de l'alumine, avec ou sans catalyseurs de démétallisation tels que le nickel, le cobalt et/ou le molybdène.

Les techniques de filtration et d'extraction par solvant sont d'autres choix qui peuvent être employés.

Le produit récupéré, notamment directement, à l'issue de cette étape d'élimination des composés non-hydratables constitue une composition ou huile raffinée qui peut être soumise ensuite à une étape de conversion en carburant d'origine naturelle, de préférence par hydrotraitement.

Etape d'hydrotraitement

Le procédé de diminution de la teneur en hétéroatomes d'une composition d'huile renouvelable selon l'invention peut en outre comprendre (E3) une étape d'hydrotraitement du produit directement issu de l'étape (E2) ou (E'2).

La composition traitée dans cette étape d'hydrotraitement est ainsi une composition raffinée, de préférence directement issue de l'un des traitements (i) à (iv).

Cette composition raffinée présente typiquement au moins une des caractéristiques suivantes, de préférence toutes :

- Une teneur en phosphore inférieure à 200ppm, de préférence inférieure à 100ppm ou à 50ppm, davantage de préférence inférieure à 3ppm,

- Une teneur en azote inférieure à 200ppm, de préférence inférieure à 100ppm, davantage de préférence inférieure à 50ppm,
- Une teneur en chlore inférieure à 50ppm, de préférence inférieure à 20ppm, davantage de préférence inférieure à 5ppm
- Une teneur en métaux inférieure à 50ppm, de préférence inférieure à 10ppm

Dans un mode de réalisation, la composition raffinée peut être hydrotraitée mélangée à une ou plusieurs coupes hydrocarbonées minérales. La ou les coupes hydrocarbonées minérales peuvent être du type naphta, kérosène ou gasoil. Cette coupe hydrocarbonée minérale peut être ajoutée dans des quantités allant de 1 à 98 % en masse ou de 1 à 95% en masse ou dans tout intervalle défini par deux de ces limites. Cette coupe hydrocarbonée minérale, qui contient des composés soufrés généralement organiques, apportera le soufre nécessaire pour maintenir l'activité catalytique du catalyseur contenant du cobalt, du nickel, du tungstène et du molybdène. En même temps, la coupe hydrocarbonée minérale est aussi au moins partiellement désulfurée.

Cette étape d'hydrotraitement est typiquement réalisée en présence de dihydrogène et d'au moins un catalyseur pour transformer les esters d'acides gras et les acides gras libres, contenus dans le produit directement issu de l'un des traitements (i) à (iv) en paraffines linéaires ou sensiblement linéaires. L'hydrotraitement peut être réalisé entre 100 et 550°C en présence de dihydrogène à des pressions allant de 0,01 à 10 MPa. Le rapport entre le dihydrogène et la charge d'alimentation peut être de 100 à 2000 Nl/l.

Cet hydrotraitement peut comprendre une ou plusieurs étapes choisies parmi l'hydrodésoxygénation, la décarboxylation et la décarbonylation.

L'hydrodésoxygénation se fait de préférence dans des réacteurs à lit fixe continu, des réacteurs à réservoir agité continu ou des réacteurs de type slurry contenant un catalyseur solide qui peut être choisi parmi les oxydes ou les sulfures de Ni, Mo, W, Co ou des mélanges comme NiW, NiMo, CoMo, NiCoW, NiCoMo, NiMoW et CoMoW comme phase catalytique, de préférence supporté sur du carbone, de l'alumine, de la silice, de l'oxyde de titane ou de la zircone.

L'hydrodésoxygénation peut être effectuée à une température de 200 à 500°C, de préférence de 220 à 400°C, sous une pression de 1 MPa à 10 MPa (10 à 100 bars), par exemple de 6 MPa, et avec un rapport dihydrogène/huile de 100 à 2000, mais de préférence de 350 à 1500, par exemple de 800 Nl $H_2$/l d'huile.

La décarboxylation et/ou la décarbonylation se fait de préférence en présence d'un catalyseur solide dans des réacteurs à cuve de type discontinu, des réacteurs à lit fixe en continu, des réacteurs à cuve agitée en continu ou des réacteurs à boue. La décarboxylation et/ou la décarbonylation peut se faire directement avec des glycérides, des esters quelconques ou avec des acides gras libres.

Le catalyseur peut être choisi parmi :

- les oxydes ou sulfures de Ni, Mo, W, Co, NiW, NiMo, CoMo, NiCoW, NiCoMo, NiMoW et CoMoW en tant que phase catalytique, de préférence supportés sur du carbone, de l'alumine, de la silice, de l'oxyde de titane ou de la zircone, ou

- des métaux ou des mélanges d'alliages du groupe 10 (Ni, Pt et Pd) et du groupe 11 (Cu et Ag) supportés par du carbone, de la magnésie, de l'oxyde de zinc, des spinelles ($Mg_2Al_2O_4$, $ZnAl_2O_4$), des pérovskites ($BaTiO_3$, $ZnTiO_3$), des calciumsilicates (comme la xonotlite), de l'alumine, de la silice ou des silices-alumines ou des mélanges de ces derniers.

Pour une performance optimale et un fonctionnement continu stable, il est préférable que le composant métallique actif du catalyseur dans le cas de Ni, Mo, W, Co ou des mélanges, soit sous forme de sulfures. Il est donc préférable que des traces de composés sulfurés décomposables (thermiquement ou catalytiquement) soient présentes ou ajoutées volontairement à la charge afin de maintenir le sulfure métallique dans son état de sulfure. À titre d'exemple, ces composés soufrés peuvent être $H_2S$, COS, $CS_2$, des mercaptans (par exemple, le méthylsulfure), des thioéthers (par exemple, le DiMéthylSulfure), des disulfures (par exemple, le DiMéthyldiSulfure), des composés thiophéniques et tétrahydrothiophéniques.

La décarboxylation et/ou décarbonylation peut également être effectuée sur des oxydes basiques, comme les oxydes de métaux alcalins, les oxydes d'alcalino-terreux, les oxydes de lanthanides, l'oxyde de zinc, les spinelles (Mg2Al2O4, ZnAl2O4), les pérovskites (BaTiO3, ZnTiO3), calciumsilicates (comme la xonotlite), soit en vrac, soit dispersés sur des supports neutres ou basiques, sur des zéolites basiques (comme les zéolites alcalines ou alcalino-terreuses à faible teneur en silice/alumine obtenues par échange ou imprégnation).

Bien que la réaction de décarboxylation et/ou décarbonylation ne nécessite pas de dihydrogène, il est préférable que la décarboxylation et/ou décarbonylation soit effectuée en présence de dihydrogène qui stabilisera l'activité catalytique en éliminant les espèces insaturées fortement adsorbées (par exemple lorsque la décarbonylation est la voie de réaction prédominante) de la surface du catalyseur par des réactions d'addition d'hydrogène. La présence de dihydrogène peut également hydrogéner les doubles liaisons présentes dans la partie acyle de l'acide gras afin d'obtenir

des produits de réaction paraffiniques à partir du processus de décarboxylation.

L'étape de décarboxylation et/ou décarbonylation peut être effectuée entre 100 et 550°C en présence de dihydrogène à des pressions allant de 0,01 à 10 MPa. Le rapport entre le dihydrogène et la charge d'alimentation peut être de 100 à 2000 Nl/l.

Système de détermination du traitement d'élimination en hétéroatomes

L'invention a également pour objet un système de détermination du traitement d'élimination en hétéroatomes à appliquer à une composition d'origine naturelle pour atteindre une teneur cible en au moins un hétéroatome, configuré, notamment programmé, pour mettre en œuvre les étapes (a) à (c) du procédé de détermination selon l'invention.

Le système de détermination comprend typiquement un ou plusieurs processeurs, par exemple un microprocesseur, un microcontrôleur ou autre. Il peut être configuré pour recevoir la teneur totale de ladite composition en au moins un hétéroatome choisi parmi le phosphore et l'azote et la teneur totale de ladite composition en composés hydratables de l'au moins un hétéroatome.

Il comprend typiquement des interfaces de sortie ou d'entrée/sortie. Il peut s'agit d'interfaces de communication sans fil (Bluetooth, WIFI ou autre) ou des connecteurs (port réseau, port USB, port série, port Firewire®, port SCSI ou autre). Ces interfaces d'entrée et/ou sortie peuvent former des moyens de communication, optionnellement bidirectionnels, entre le système de détermination et par exemple une interface utilisateur, permettant notamment au système de recevoir les teneurs relatives à la composition.

Le système de détermination peut également comprendre des moyens de stockage qui peuvent être une mémoire vive (RAM), une mémoire morte programmable effaçable électriquement (EEPROM), une mémoire flash, une mémoire externe ou autre. Ces moyens de stockage peuvent, entre autres, stocker des données reçues, les valeurs mesurées, les valeurs calculées, une base de données et/ou un modèle, et un ou plusieurs programmes informatiques.

Le système peut ainsi comprendre au moins un processeur configuré pour :

(a) recevoir la teneur totale de ladite composition en au moins un hétéroatome choisi parmi le phosphore et l'azote, recevoir la teneur totale de ladite composition en composés hydratables de l'au moins un hétéroatome et déterminer en tant que paramètre représentatif de la qualité de la composition, le rapport de la teneur totale en l'au moins un hétéroatome présent dans les composés de cet hétéroatome hydratables à pH neutre identifiés sur la teneur totale de la composition en cet hétéroatome, ce rapport étant déterminé pour le phosphore, pour l'azote ou pour chacun de ces deux hétéroatomes,

(b) estimer une teneur résiduelle en au moins un hétéroatome choisi parmi le phosphore et l'azote, à partir du paramètre représentatif, cette teneur résiduelle correspondant à la teneur en l'au moins un hétéroatome après élimination des composés hydratables à pH=7 contenant cet hétéroatome,

(c) déterminer un traitement à appliquer à ladite composition pour atteindre une teneur cible en l'au moins un hétéroatome en sortie du traitement, ce traitement étant choisi parmi :

(i) un lavage avec de l'eau à pH neutre,

(ii) un lavage avec de l'eau à pH neutre suivi d'une étape d'élimination de composés non hydratables,

(iii) un lavage avec de l'eau à pH neutre suivi d'une étape d'élimination de composés hydratables à un pH autre qu'un pH neutre,

(iv) un lavage avec de l'eau à pH neutre suivi d'une étape d'élimination de composés hydratables à un pH autre qu'un pH neutre puis d'une étape d'élimination de composés non hydratables,

l'au moins un processeur étant configuré pour :

comparer la dite teneur résiduelle à la teneur cible et

choisir le traitement (i) si la teneur résiduelle est inférieure ou égale à la teneur cible,

sinon, utiliser une base de données ou un modèle configuré pour fournir, une estimation d'une teneur en l'au moins un hétéroatome en sortie de chaque traitement (ii) à (iv) en fonction d'une teneur en cet hétéroatome en entrée du traitement égale à la teneur résiduelle, et choisir le traitement pour lequel la teneur en sortie estimée est inférieure ou

égale à la teneur cible, et optionnellement pour lequel le nombre d'étapes de traitement est le plus faible.

L'au moins un processeur peut en outre être configuré pour :

(c) si aucune des teneurs en sortie estimées n'est inférieure ou égal à la teneur cible, réitérer les étapes (a) à (c) après dilution de ladite composition d'origine naturelle avec une autre composition d'origine naturelle, optionnellement une composition présentant un paramètre représentatif de la qualité de la composition supérieur à celui de ladite composition,
et/ou
(c) choisir un traitement et les conditions opératoires de ce traitement pour lequel la teneur en sortie estimée est inférieure ou égale à la teneur cible, et optionnellement pour lequel le nombre d'étapes de traitement est le plus faible et/ou pour lequel la quantité de réactif utilisée est la plus faible, la base de données ou le modèle étant configuré pour fournir une estimation, pour chaque traitement (ii) à (iv), des teneurs en l'au moins un hétéroatome en sortie dudit traitement, obtenue pour différentes conditions opératoires des traitements (i) à (iv)

Dans tous les cas, l'au moins un processeur peut recevoir les données via une ou plusieurs interfaces d'entrée, ou d'entrée et sortie, notamment du type précité, et peut stocker une base de données ou un modèle dans des moyens de stockage, notamment du type précité.

## Figures

D'autres particularités et avantages de l'invention ressortiront à la lecture de la description faite ci-après d'un mode de réalisation particulier de l'invention, donné à titre indicatif mais non limitatif, en référence aux dessins annexés sur lesquels :

La figure 1 représente schématiquement un exemple de mise en œuvre du procédé de traitement selon l'invention.

La figure 2 représente schématiquement une enceinte de lavage.

La figure 3 est un tableau représentant les formules chimiques des phospholipides dans l'eau en fonction du pH, le groupement R représente un di-glycéride.

La figure 1 représente schématiquement un procédé de diminution de la teneur en hétéroatomes d'une composition d'origine naturelle H.
La composition d'origine naturelle H à traiter est d'abord soumise à l'étape (a) de détermination d'un paramètre Q représentatif de sa qualité, ici le paramètre relatif à la teneur en phosphore.
Ce paramètre Q peut s'écrire (équation 1) :
[Math 1]

$$Q = \frac{\sum[PC, Sels\ P; PI]}{[P]} \text{ (équation 1)}$$

où

$\Sigma$[*PC, Sels P; PI*] représente la teneur totale de la composition H en phosphore présent dans les composés du phosphore hydratables à pH neutre, à savoir les sels de phosphore hydratables, PC et PI,

et [P] représente la teneur totale en phosphore (élémentaire) de la composition H.

Au cours de l'étape (b) on estime la teneur résiduelle en phosphore, notée [P]r, à partir du paramètre Q. Cette teneur résiduelle correspond à la teneur totale en phosphore à laquelle on soustrait la teneur en phosphore présent dans les composés du phosphore hydratables à pH neutre et peut donc être calculée de la manière suivante (équation 2) :
[Math 2]

$$[P]r = [P] \times (1 - Q) \text{(équation 2)}$$

Lors de l'étape (c), on compare ensuite cette teneur résiduelle [P]r à la teneur cible (notée [P]c) souhaitée et on

choisit l'un des traitements (i) à (iv) tel que précédemment décrit, et l'on soumet la composition au traitement retenu ce qui permet d'obtenir une composition raffinée.

Ainsi, tel que représenté sur la figure 1 :

si [P]r < [P]c, seule l'étape de lavage à l'eau à pH neutre E1(a) est mise en œuvre avant l'étape d'hydrotraitement E3,

Si [P]r > [P]c, on pourra mettre en œuvre, avant l'étape d'hydrotraitement E3, la succession des étapes E1(a) et E1(c) (élimination des composés non hydratables), la succession des étapes E1(a), E1(b) (élimination des composés hydratables à un pH autre qu'un pH neutre), ou encore la succession des étapes E1(a), E1(b), E1(c).

La figure 2 représente une enceinte 1, par exemple un bac de stockage ou une capacité de dessalage contenant la composition H. Cette enceinte 1 est équipée d'un circuit de recirculation 2 pourvue d'une pompe 3. En amont de la pompe 3 par rapport au sens de recirculation du circuit de recirculation 2, une conduite 4 permet d'injecter de l'eau dans le circuit 2. Une conduite de soutirage 5 située en fond de l'enceinte 1 permet de récupérer l'huile après lavage à l'eau.

## Exemples:

### Exemple 1 : Analyse de la teneur en phosphore de différentes compositions d'origine naturelle

Le tableau 1 rassemble les résultats de l'analyse de la phase organique et de la phase aqueuse récupérée après une extraction liquide/liquide de différentes compositions avec une solution aqueuse à pH = 7 contenant de l'EDTA.

Les mesures de RMN du $^{31}P$ dans la phase organique ont été réalisées dans du chloroforme deutéré ($CDCl_3$) et celles réalisées dans la phase aqueuse ont été réalisées dans un mélange d'eau deutérée ($D_2O$) et d'alcool méthylique (MeOH), par exemple selon le protocole décrit ci-après.

Une quantité précise d'échantillon d'huile renouvelable est prélevée dans un vial auquel on ajoute une quantité précise d'un standard phosphoré soluble en phase organique et d'un standard phosphoré soluble en phase aqueuse. Cet échantillon est ensuite solubilisé dans du chloroforme deutéré. On y ajoute ensuite une solution de 0,2 M d'EDTA à pH7 en MeOH/$D_2O$ afin de maximiser la résolution analytique, en s'inspirant des articles de la littérature : T. Glonek, M. Lunde, M. Mudgett, T. C. Myers (1971) Studies of Biological Polyphosphate Through the Use of Phosphorus-31 Nuclear Magnetic Resonance. In : Archives of Biochemistry and Biophysics, vol. 142, p. 508-513 ; T. O. Hendersen, T. Glonek, T. C. Myers (1974) Phosphorus-31 Nuclear Magnetic Resonance Spectroscopy of Phospholipids. In : Biochemistry, vol. 13, n° 3, p. 623-628. Les phases aqueuses et organiques sont ensuite séparées par décantation pendant une nuit et analysées séparément par RMN $^{31}P$. L'identification des espèces se fait grâce à leur déplacement chimique par comparaison avec des standards de structure équivalente et leur quantification se fait par ratio de l'intensité de leur signal $^{31}P$ sur l'intensité du standard soluble dans la même phase introduit initialement dans l'échantillon.

La composition C1 est une graisse de volaille, la composition C2 est un mélange de graisses provenant de plusieurs animaux, la composition C3 est de la graisse de porc.

[Table 1]

| **Composition** | | | **C1** | **C2** | **C3** |
|---|---|---|---|---|---|
| | | **Unité** | | | |
| Analyse élémentaire de la composition par XRF | Teneur en P | Ppm (élément $^{31}P$) | 500 | 132 | 751 |
| Analyse par RMN $^{31}P$ de la phase organique | Teneur en P présent dans PC | Ppm (élément $^{31}P$) | **87** | **32** | **42** |
| | Teneur en P présent dans PI | Ppm (élément $^{31}P$) | **ND** | **8** | **8** |
| | Teneur en P présent dans les autres composés contenant du phosphore | Ppm (élément $^{31}P$) | 233 | 35 | 32 |
| | Teneur totale en P présent dans les composés organiques contenant du phosphore | Ppm (élément $^{31}P$) | 320 | 75 | 82 |

(suite)

| Composition | | | C1 | C2 | C3 |
|---|---|---|---|---|---|
| | | Unité | | | |
| Analyse par RMN $^{31}P$ de la phase aqueuse | Teneur en P présent dans les phosphates | Ppm (élément $^{31}P$) | **165** | **35** | **421** |
| | Teneur en P présent dans les autres composés phosphorés | Ppm (élément $^{31}P$) | **ND** | **ND** | **20** |
| Teneur totale en phosphore de la composition obtenue par analyse RMN | Teneur en P | Ppm (élément $^{31}P$) | 485 | 110 | 524 |
| Teneur totale en P présent dans les composés du P hydratables à pH 7 | Teneur en P | Ppm (élément $^{31}P$) | 252 | 75 | 491 |
| Paramètre Q | | | 0,50 | 0,57 | 0,65 |

Les données du tableau 1 permettent de calculer la teneur totale des composés du phosphore hydratables à pH 7 (correspondant à la somme des teneurs en P provenant de PC et PI et de la phase aqueuse- les valeurs additionnées sont en gras dans le tableau) et le paramètre Q représentatif de la qualité de chacune des compositions calculé en divisant la teneur totale en P présent dans les composés du P hydratables à pH 7 par la teneur en P totale de la composition mesurée par XRF.

Exemple 2 - Lavage à l'eau à pH neutre

La composition C3 de l'exemple 1 a été soumise à un lavage à l'eau à pH neutre avec des ratios eau / graisse de 4, 8 et 12.

L'eau et la graisse ont été séparés par centrifugation à 4800 g avant mesure de la teneur en phosphore de la phase huileuse.

Le tableau 2 rassemble les teneurs en phosphore de la phase huileuse mesurées par XRF pour les différents ratios testés.

[Table 2]

| Traitement composition C3 | Teneur en phosphore (ppm) | Ratio eau/graisse (% m) | Teneur en phosphore de la phase huileuse (ppm) |
|---|---|---|---|
| Aucun traitement | 751 | - | - |
| Lavage à l'eau | | 4 | 7.2 |
| | | 8 | 7.8 |
| | | 12 | 6.6 |

On notera qu'un abattement important du phosphore est observé dès un lavage à l'eau avec 4%m d'eau. Ainsi, pour une composition de graisse présentant un paramètre représentatif de la qualité de la composition C3 de 0,65, une étape de lavage à l'eau permet d'éliminer la quasi-totalité du phosphore présent dans la composition, laquelle pourrait être ensuite éventuellement traitée directement dans une unité d'hydrotraitement afin de produire un carburant en fonction de la spécification en phosphore des charges entrant dans cette unité d'hydrotraitement.

**Revendications**

1. Méthode de détermination d'un paramètre représentatif de la qualité d'une composition d'origine naturelle comprenant des hétéroatomes et des lipides choisis parmi des lipides phénoliques, des acides gras, des triglycérides, des diglycérides, des mono-glycérides, des phospholipides, des esters d'acides gras et/ou un mélange de deux ou plusieurs de ces composés, la méthode comprenant :

(A) la détermination de la teneur totale de ladite composition en au moins un hétéroatome choisi parmi le phosphore et l'azote,
(B) l'identification des composés de l'au moins un hétéroatome hydratables à pH neutre et la détermination de la teneur totale de ladite composition en l'au moins un hétéroatome présent dans les composés de cet hétéroatome hydratables à pH neutre identifiés,
(C) la détermination en tant que paramètre représentatif de la qualité de la composition du rapport de la teneur totale en l'au moins un hétéroatome présent dans les composés de cet hétéroatome hydratables à pH neutre identifiés sur la teneur totale de la composition en cet hétéroatome, ce rapport étant déterminé pour le phosphore, pour l'azote ou pour chacun de ces deux hétéroatomes.

**2.** Méthode de détermination selon la revendication 1, dans laquelle l'étape (B) est réalisée au moyen d'une technique d'analyse choisie parmi la RMN, la spectroscopie de masse, la chromatographie ionique, la chromatographie en phase liquide couplée à la spectrométrie de masse et la chromatographie sur couche mince performante.

**3.** Méthode de détermination selon la revendication 1 ou 2, dans laquelle ladite composition d'origine naturelle contient une ou plusieurs huiles choisie parmi une huile végétale, une huile ou une graisse animale, une huile usagée, une huile produite par des microorganismes, des esters résultant de la trans-estérification des esters d'acides gras contenus dans une ou plusieurs de ces huiles, ainsi que leurs mélanges.

**4.** Procédé de détermination du traitement d'élimination en hétéroatomes à appliquer à une composition d'origine naturelle comprenant des hétéroatomes et des lipides choisis parmi des lipides phénoliques, des acides gras, des triglycérides, des di-glycérides, des mono-glycérides, des phospholipides, des esters d'acides gras et/ou un mélange de deux ou plusieurs de ces composés pour atteindre une teneur cible pour au moins un hétéroatome, le procédé comprenant :

(a) une étape de détermination d'au moins un paramètre représentatif de la qualité de la composition au moyen de la méthode de détermination selon l'une quelconque des revendications 1 à 3,
(b) une étape d'estimation d'une teneur résiduelle en au moins un hétéroatome choisi parmi le phosphore et l'azote, à partir du paramètre représentatif, cette teneur résiduelle correspondant à la teneur en l'au moins un hétéroatome après élimination des composés hydratables à pH=7 contenant cet hétéroatome,
(c) une étape de détermination d'un traitement à appliquer à ladite composition pour atteindre une teneur cible en l'au moins un hétéroatome en sortie du traitement, ledit traitement étant choisi parmi :

(i) un lavage avec de l'eau à pH neutre,
(ii) un lavage avec de l'eau à pH neutre suivi d'une étape d'élimination de composés non hydratables,
(iii) un lavage avec de l'eau à pH neutre suivi d'une étape d'élimination de composés hydratables à un pH autre qu'un pH neutre,
(iv) un lavage avec de l'eau à pH neutre suivi d'une étape d'élimination de composés hydratables à un pH autre qu'un pH neutre puis d'une étape d'élimination de composés non hydratables,

dans laquelle

on compare la dite teneur résiduelle à la teneur cible et
on choisit le traitement (i) si la teneur résiduelle est inférieure ou égale à la teneur cible,
sinon, on utilise une base de données ou un modèle configuré pour fournir, une estimation d'une teneur en l'au moins un hétéroatome en sortie de chaque traitement (ii) à (iv) en fonction d'une teneur en cet hétéroatome en entrée du traitement égale à la teneur résiduelle, et on choisit le traitement pour lequel la teneur en sortie estimée est inférieure ou égale à la teneur cible, et optionnellement pour lequel le nombre d'étapes de traitement est le plus faible.

**5.** Procédé de détermination selon la revendication 4, dans lequel, au cours de l'étape (c), si aucune des teneurs en sortie estimées n'est inférieure ou égal à la teneur cible, on réitère les étapes (a) à (c) en diluant ladite composition d'origine naturelle avec une autre composition d'origine naturelle, optionnellement une composition présentant un paramètre représentatif de la qualité de la composition supérieur à celui de ladite composition.

**6.** Procédé de détermination selon la revendication 4 ou 5, dans lequel, la base de données ou le modèle est configuré pour fournir une estimation, pour chaque traitement (ii) à (iv), des teneurs en l'au moins un hétéroatome en sortie dudit traitement, obtenue pour différentes conditions opératoires des traitements (i) à (iv) et au cours de l'étape (c), on

choisit un traitement et les conditions opératoires de ce traitement pour lequel la teneur en sortie estimée est inférieure ou égale à la teneur cible, et optionnellement pour lequel le nombre d'étapes de traitement est le plus faible et/ou pour lequel la quantité de réactif utilisée est la plus faible.

**7.** Procédé de détermination selon l'une quelconque des revendications 4 à 6, dans lequel ladite composition d'origine naturelle contient une ou plusieurs huiles choisie parmi une huile végétale, une huile ou une graisse animale, une huile usagée, une huile produite par des microorganismes, des esters résultant de la trans-estérification des esters d'acides gras contenus dans une ou plusieurs de ces huiles, ainsi que leurs mélanges.

**8.** Procédé de diminution de la teneur en hétéroatomes d'une composition d'origine naturelle comprenant des hétéroatomes et des lipides choisis parmi des lipides phénoliques, des acides gras, des triglycérides, des di-glycérides, des mono-glycérides, des phospholipides, des esters d'acides gras et/ou un mélange de deux ou plusieurs de ces composés, le procédé comprenant :

(E1) une étape de détermination du traitement d'élimination en hétéroatomes à appliquer à la composition d'origine naturelle pour atteindre une teneur cible en au moins un hétéroatome, au moyen du procédé de détermination selon l'une quelconque des revendications 4 à 7, et
(E2) une étape au cours de laquelle on soumet ladite composition d'origine naturelle au traitement déterminé à l'étape (E1).

**9.** Procédé de diminution de la teneur en hétéroatomes d'une composition d'origine naturelle comprenant des hétéroatomes et des lipides choisis parmi des lipides phénoliques, des acides gras, des triglycérides, des di-glycérides, des mono-glycérides, des phospholipides, des esters d'acides gras et/ou un mélange de deux ou plusieurs de ces composés, le procédé comprenant :

(E'2) une étape d'élimination en hétéroatomes au cours de laquelle on soumet la composition d'origine naturelle à un traitement choisi parmi :

(i) un lavage avec de l'eau à pH neutre,
(ii) un lavage avec de l'eau à pH neutre suivi d'une étape d'élimination de composés non hydratables,
(iii) un lavage avec de l'eau à pH neutre suivi d'une étape d'élimination de composés hydratables à un pH autre qu'un pH neutre,
(iv) un lavage avec de l'eau à pH neutre suivi d'une étape d'élimination de composés hydratables à un pH autre qu'un pH neutre puis d'une étape d'élimination de composés non hydratables,

et dans lequel, la composition d'origine naturelle présente un paramètre représentatif de la qualité de la composition, préalablement déterminé au moyen de la méthode de détermination selon l'une quelconque des revendications 1 à 3, qui est supérieur ou égal à un seuil au-delà duquel l'efficacité de l'étape d'élimination est suffisante pour réduire la teneur en hétéroatomes de ladite composition à une teneur cible ou inférieure à une teneur cible.

**10.** Procédé de diminution selon la revendication 8 ou 9, comprenant en outre (E3) une étape d'hydrotraitement du produit directement issu de l'étape (E2) ou (E'2).

**11.** Procédé de diminution selon l'une quelconque des revendications 8 à 10, dans lequel la composition d'origine naturelle contient une ou plusieurs huiles choisie parmi une huile végétale, une huile ou une graisse animale, une huile usagée, une huile produite par des microorganismes, des esters résultant de la trans-estérification des esters d'acides gras contenus dans une ou plusieurs de ces huiles, ainsi que leurs mélanges.

**12.** Procédé de diminution selon l'une quelconque des revendications 8 à 11, dans lequel, lors de l'étape (E2) ou (E'2), le lavage avec de l'eau à pH neutre de chacun des traitements (i) à (iv) est mis en œuvre avec un ratio en masse eau /composition de 1 :99 à 10 : 90.

**13.** Procédé de diminution selon l'une quelconque des revendications 8 à 12, dans lequel, lors de l'étape (E2) ou (E'2), le lavage avec de l'eau à pH neutre de chacun des traitements (i) à (iv) est mis en œuvre dans une enceinte choisie parmi un bac de stockage, une capacité de dessalage, un ballon.

**14.** Système de détermination du traitement d'élimination en hétéroatomes à appliquer à une composition d'origine

naturelle pour atteindre une teneur cible en au moins un hétéroatome, configuré pour mettre en œuvre les étapes (a) à (c) du procédé de détermination selon l'une quelconque des revendications 4 à 6, le système comprenant au moins un processeur configuré pour:

(a) recevoir la teneur totale de ladite composition en au moins un hétéroatome choisi parmi le phosphore et l'azote, et la teneur totale de ladite composition en composés hydratables de l'au moins un hétéroatome et pour déterminer en tant que paramètre représentatif de la qualité de la composition, le rapport de la teneur totale en l'au moins un hétéroatome présent dans les composés de cet hétéroatome hydratables à pH neutre identifiés sur la teneur totale de la composition en cet hétéroatome, ce rapport étant déterminé pour le phosphore, pour l'azote ou pour chacun de ces deux hétéroatomes,
(b) estimer une teneur résiduelle en au moins un hétéroatome choisi parmi le phosphore et l'azote, à partir du paramètre représentatif, cette teneur résiduelle correspondant à la teneur en l'au moins un hétéroatome après élimination des composés hydratables à pH=7 contenant cet hétéroatome,
(c) déterminer un traitement à appliquer à ladite composition pour atteindre une teneur cible en l'au moins un hétéroatome en sortie du traitement, ce traitement étant choisi parmi :

(i) un lavage avec de l'eau à pH neutre,
(ii) un lavage avec de l'eau à pH neutre suivi d'une étape d'élimination de composés non hydratables,
(iii) un lavage avec de l'eau à pH neutre suivi d'une étape d'élimination de composés hydratables à un pH autre qu'un pH neutre,
(iv) un lavage avec de l'eau à pH neutre suivi d'une étape d'élimination de composés hydratables à un pH autre qu'un pH neutre puis d'une étape d'élimination de composés non hydratables,

l'au moins un processeur étant configuré pour :

comparer la dite teneur résiduelle à la teneur cible et
choisir le traitement (i) si la teneur résiduelle est inférieure ou égale à la teneur cible,
sinon, utiliser une base de données ou un modèle configuré pour fournir, une estimation d'une teneur en l'au moins un hétéroatome en sortie de chaque traitement (ii) à (iv) en fonction d'une teneur en cet hétéroatome en entrée du traitement égale à la teneur résiduelle, et choisir le traitement pour lequel la teneur en sortie estimée est inférieure ou égale à la teneur cible, et optionnellement pour lequel le nombre d'étapes de traitement est le plus faible.

**15.** Système de détermination selon la revendication 14, dans lequel l'au moins un processeur est en outre configuré pour :

(c) si aucune des teneurs en sortie estimées n'est inférieure ou égal à la teneur cible, réitérer les étapes (a) à (c) après dilution de ladite composition d'origine naturelle avec une autre composition d'origine naturelle, optionnellement une composition présentant un paramètre représentatif de la qualité de la composition supérieur à celui de ladite composition, et/ou
(c) choisir un traitement et les conditions opératoires de ce traitement pour lequel la teneur en sortie estimée est inférieure ou égale à la teneur cible, et optionnellement pour lequel le nombre d'étapes de traitement est le plus faible et/ou pour lequel la quantité de réactif utilisée est la plus faible, la base de données ou le modèle étant configuré pour fournir une estimation, pour chaque traitement (ii) à (iv), des teneurs en l'au moins un hétéroatome en sortie dudit traitement, obtenue pour différentes conditions opératoires des traitements (i) à (iv).

## Patentansprüche

**1.** Methode zur Bestimmung eines Parameters, der repräsentativ für die Qualität einer Zusammensetzung natürlichen Ursprungs ist, die Heteroatome und Lipide umfasst, die aus phenolischen Lipiden, Fettsäuren, Triglyceriden, Diglyceriden, Monoglyceriden, Phospholipiden, Fettsäureestern und/oder einer Mischung aus zwei oder mehreren dieser Verbindungen ausgewählt sind, wobei die Methode Folgendes umfasst:

(A) die Bestimmung des Gesamtgehalts der genannten Zusammensetzung an mindestens einem aus Phosphor und Stickstoff ausgewählten Heteroatom,
(B) die Identifizierung der Verbindungen des mindestens einen Heteroatoms, die bei neutralem pH-Wert hydratisierbar sind, und die Bestimmung des Gesamtgehalts der genannten Zusammensetzung an dem

mindestens einen Heteroatom, das in den identifizierten, bei neutralem pH-Wert hydratisierbaren Verbindungen dieses Heteroatoms vorhanden ist,

(C) die Bestimmung, als repräsentativer Parameter für die Qualität der Zusammensetzung, des Verhältnisses des Gesamtgehalts an dem mindestens einen Heteroatom, das in den identifizierten, bei neutralem pH-Wert hydratisierbaren Verbindungen dieses Heteroatoms vorhanden ist, zum Gesamtgehalt der Zusammensetzung an diesem Heteroatom, wobei dieses Verhältnis für Phosphor, für Stickstoff oder für jedes dieser beiden Heteroatome bestimmt wird.

**2.** Bestimmungsmethode nach Anspruch 1, wobei Schritt (B) mittels einer Analysetechnik durchgeführt wird, die aus NMR, Massenspektroskopie, Ionenchromatographie, Flüssigchromatographie gekoppelt mit Massenspektrometrie und Hochleistungsdünnschichtchromatographie ausgewählt ist.

**3.** Bestimmungsmethode nach Anspruch 1 oder 2, wobei die genannte Zusammensetzung natürlichen Ursprungs ein oder mehrere Öle enthält, das aus einem Pflanzenöl, einem tierischen Öl oder Fett, einem Altöl, einem durch Mikroorganismen erzeugten Öl, Estern, die aus der Umesterung der in einem oder mehreren dieser Öle enthaltenen Fettsäureester resultieren, sowie deren Mischungen ausgewählt ist.

**4.** Verfahren zur Bestimmung einer Behandlung zur Heteroatomentfernung, die auf eine Zusammensetzung natürlichen Ursprungs anzuwenden ist, die aus phenolischen Lipiden, Fettsäuren, Triglyceriden, Diglyceriden, Monoglyceriden, Phospholipiden, Fettsäureestern und/oder einer Mischung aus zwei oder mehr dieser Verbindungen ausgewählte Heteroatome und Lipide umfasst, um einen Zielgehalt für mindestens ein Heteroatom zu erreichen, wobei das Verfahren Folgendes umfasst:

(a) einen Schritt zur Bestimmung mindestens eines für die Qualität der Zusammensetzung repräsentativen Parameters mittels der Bestimmungsmethode nach einem der Ansprüche 1 bis 3,

(b) einen Schritt zur Schätzung eines Restgehalts an mindestens einem aus Stickstoff und Phosphor ausgewählten Heteroatom, ausgehend vom repräsentativen Parameter, wobei dieser Restgehalt dem Gehalt an mindestens einem Heteroatom nach Entfernung der bei einem pH-Wert von 7 hydratisierbaren Verbindungen, die dieses Heteroatom enthalten, entspricht,

(c) einen Schritt zur Bestimmung einer Behandlung, die auf die genannte Zusammensetzung anzuwenden ist, um am Ende der Behandlung einen Zielgehalt an dem mindestens einen Heteroatom zu erreichen, wobei die genannte Behandlung ausgewählt ist aus:

(i) einem Waschen mit pH-neutralem Wasser,

(ii) einem Waschen mit pH-neutralem Wasser, gefolgt von einem Schritt zur Entfernung nicht hydratisierbarer Verbindungen,

(iii) einem Waschen mit pH-neutralem Wasser, gefolgt von einem Schritt zur Entfernung von Verbindungen, die bei einem anderen pH-Wert als einem neutralen pH-Wert hydratisierbar sind,

(iv) einem Waschen mit pH-neutralem Wasser, gefolgt von einem Schritt zur Entfernung von Verbindungen, die bei einem anderen pH-Wert als einem neutralen pH-Wert hydratisierbar sind, und dann einem Schritt zur Entfernung nicht hydratisierbarer Verbindungen,

wobei

der genannte Restgehalt mit dem Zielgehalt verglichen wird und

die Behandlung (i) gewählt wird, wenn der Restgehalt kleiner oder gleich dem Zielgehalt ist,

andernfalls eine Datenbank oder ein Modell verwendet wird, das konfiguriert ist, um eine Schätzung des Gehalts an dem mindestens einem Heteroatom am Ende jeder Behandlung (ii) bis (iv) in Abhängigkeit vom Gehalt an diesem Heteroatom am Anfang der Behandlung, der gleich dem Restgehalt ist, bereitzustellen, und die Behandlung ausgewählt wird, bei welcher der geschätzte Gehalt am Ende der Behandlung kleiner oder gleich dem Zielgehalt ist und bei welcher optional die Anzahl der Behandlungsschritte am geringsten ist.

**5.** Bestimmungsverfahren nach Anspruch 4, wobei im Verlauf des Schritts (c), wenn keiner der geschätzten Gehalte am Ende kleiner oder gleich dem Zielgehalt ist, die Schritte (a) bis (c) wiederholt werden, indem die genannte Zusammensetzung natürlichen Ursprungs mit einer anderen Zusammensetzung natürlichen Ursprungs verdünnt wird, optional mit einer Zusammensetzung, die einen für die Qualität der Zusammensetzung repräsentativen Parameter aufweist, der höher als derjenige der genannten Zusammensetzung ist.

6. Bestimmungsverfahren nach Anspruch 4 oder 5, wobei die Datenbank oder das Modell konfiguriert ist, um für jede Behandlung (ii) bis (iv) eine für verschiedene Betriebsbedingungen der Behandlungen (i) bis (iv) erhaltene Schätzung der Gehalte an dem mindestens einen Heteroatom am Ende der genannten Behandlung bereitzustellen, und im Verlauf des Schritts (c) eine Behandlung und die Betriebsbedingungen für diese Behandlung ausgewählt werden, bei welcher der geschätzte Endgehalt kleiner oder gleich dem Zielgehalt ist, und optional bei welcher die Anzahl der Behandlungsschritte am geringsten ist und/oder bei welcher die Menge an verwendetem Reagenz am geringsten ist.

7. Bestimmungsverfahren nach einem der Ansprüche 4 bis 6, wobei die genannte Zusammensetzung natürlichen Ursprungs ein oder mehrere Öle enthält, die aus einem Pflanzenöl, einem tierischen Öl oder Fett, einem Altöl, einem durch Mikroorganismen erzeugten Öl, Estern, die aus der Umesterung der in einem oder mehreren dieser Öle enthaltenen Fettsäureester resultieren, sowie deren Mischungen ausgewählt sind.

8. Verfahren zur Verringerung des Heteroatomgehalts einer Zusammensetzung natürlichen Ursprungs, die aus phenolischen Lipiden, Fettsäuren, Triglyceriden, Diglyceriden, Monoglyceriden, Phospholipiden, Fettsäureestern und/oder einer Mischung aus zwei oder mehreren dieser Verbindungen ausgewählte Heteroatome und Lipide umfasst, wobei das Verfahren Folgendes umfasst:

   (E1) einen Schritt zur Bestimmung der Behandlung zur Heteroatomentfernung, die auf die Zusammensetzung natürlichen Ursprungs anzuwenden ist, um einen Zielgehalt an mindestens einem Heteroatom zu erreichen, mittels des Bestimmungsverfahrens nach einem der Ansprüche 4 bis 7, und
   (E2) einen Schritt, im Verlauf dessen die Zusammensetzung natürlichen Ursprungs der in Schritt (E1) bestimmten Behandlung unterzogen wird.

9. Verfahren zur Verringerung des Heteroatomgehalts einer Zusammensetzung natürlichen Ursprungs, die aus phenolischen Lipiden, Fettsäuren, Triglyceriden, Diglyceriden, Monoglycerid, Phospholipid, Fettsäureester und/oder einer Mischung aus zwei oder mehreren dieser Verbindungen ausgewählte Heteroatome und Lipide umfasst, wobei das Verfahren Folgendes umfasst:

   (E'2) einem Schritt zur Heteroatomentfernung, im Verlauf dessen die Zusammensetzung natürlichen Ursprungs einer Behandlung unterzogen wird, die ausgewählt ist aus:

      (i) einem Waschen mit pH-neutralem Wasser,
      (ii) einem Waschen mit pH-neutralem Wasser, gefolgt von einem Schritt zur Entfernung nicht hydratisierbarer Verbindungen,
      (iii) einem Waschen mit pH-neutralem Wasser, gefolgt von einem Schritt zur Entfernung von Verbindungen, die bei einem anderen pH-Wert als einem neutralen pH-Wert hydratisierbar sind,
      (iv) einem Waschen mit pH-neutralem Wasser, gefolgt von einem Schritt zur Entfernung von Verbindungen, die bei einem anderen pH-Wert als einem neutralen pH-Wert hydratisierbar sind, und dann von einem Schritt zur Entfernung nicht hydratisierbarer Verbindungen,

   und wobei die Zusammensetzung natürlichen Ursprungs einen für die Qualität der Zusammensetzung repräsentativen Parameter aufweist, der zuvor mittels des Bestimmungsverfahrens nach einem der Ansprüche 1 bis 3 bestimmt wurde und größer oder gleich einem Schwellenwert ist, oberhalb dessen die Wirksamkeit des Entfernungsschritts ausreicht, um den Heteroatomgehalt der Zusammensetzung auf einen Zielgehalt oder unter einen Zielgehalt zu reduzieren.

10. Verringerungsverfahren nach Anspruch 8 oder 9, das ferner (E3) einen Schritt zur Hydrobehandlung des unmittelbar aus Schritt (E2) oder (E'2) stammenden Produkts umfasst.

11. Verringerungsverfahren nach einem der Ansprüche 8 bis 10, wobei die Zusammensetzung natürlichen Ursprungs ein oder mehrere Öle enthält, die aus einem Pflanzenöl, einem tierischen Öl oder Fett, einem Altöl, einem durch Mikroorganismen erzeugten Öl, Estern, die aus der Umesterung der in einem oder mehreren dieser Öle enthaltenen Fettsäureester resultieren, sowie deren Mischungen ausgewählt sind.

12. Verringerungsverfahren nach einem der Ansprüche 8 bis 11, wobei im Verlauf des Schritts (E2) oder (E'2) das Waschen mit pH-neutralem Wasser bei jeder der Behandlungen (i) bis (iv) mit einem Massenverhältnis von Wasser zu Zusammensetzung von 1:99 bis 10:90 durchgeführt wird.

**13.** Verringerungsverfahren nach einem der Ansprüche 8 bis 12, wobei im Verlauf des Schritts (E2) oder (E'2) das Waschen mit pH-neutralem Wasser bei jeder der Behandlungen (i) bis (iv) in einem Behälter durchgeführt wird, der aus einem Lagertank, einem Entsalzungsbehälter oder einem Kugelbehälter ausgewählt ist.

**14.** System zur Bestimmung der auf eine Zusammensetzung natürlichen Ursprungs anzuwendenden Behandlung zur Heteroatomentfernung, um einen Zielgehalt an mindestens einem Heteroatom zu erreichen, das konfiguriert ist, um die Schritte (a) bis (c) des Bestimmungsverfahrens nach einem der Ansprüche 4 bis 6 durchzuführen, wobei das System mindestens einen Prozessor umfasst, der konfiguriert ist, um:

(a) den Gesamtgehalt der genannten Zusammensetzung an mindestens einem aus Phosphor und Stickstoff ausgewählten Heteroatom und den Gesamtgehalt der Zusammensetzung an hydratisierbaren Verbindungen des mindestens einen Heteroatoms aufzunehmen, und um als repräsentativen Parameter für die Qualität der Zusammensetzung das Verhältnis des Gesamtgehalts an dem mindestens einen Heteroatom, das in den identifizierten, bei neutralem pH-Wert hydratisierbaren Verbindungen dieses Heteroatoms vorhanden ist, zum Gesamtgehalt der Zusammensetzung an diesem Heteroatom zu bestimmen, wobei dieses Verhältnis für Phosphor, für Stickstoff oder für jedes dieser beiden Heteroatome bestimmt wird,
(b) einen Restgehalt an mindestens einem aus Stickstoff und Phosphor ausgewählten Heteroatom zu schätzen, ausgehend vom repräsentativen Parameter, wobei dieser Restgehalt dem Gehalt an mindestens einem Heteroatom nach Entfernung der bei einem pH-Wert von 7 hydratisierbaren Verbindungen entspricht, die dieses Heteroatom enthalten,
(c) eine Behandlung, die auf die genannte Zusammensetzung anzuwenden ist, zu bestimmen, um am Ende der Behandlung einen Zielgehalt an dem mindestens einen Heteroatom zu erreichen, wobei diese Behandlung ausgewählt ist aus:

(i) einem Waschen mit pH-neutralem Wasser,
(ii) einem Waschen mit pH-neutralem Wasser, gefolgt von einem Schritt zur Entfernung nicht hydratisierbarer Verbindungen,
(iii) einem Waschen mit pH-neutralem Wasser, gefolgt von einem Schritt zur Entfernung von Verbindungen, die bei einem anderen pH-Wert als einem neutralen pH-Wert hydratisierbar sind,
(iv) einem Waschen mit pH-neutralem Wasser, gefolgt von einem Schritt zur Entfernung von Verbindungen, die bei einem anderen pH-Wert als einem neutralen pH-Wert hydratisierbar sind, und dann einem Schritt zur Entfernung nicht hydratisierbarer Verbindungen,

wobei der mindestens eine Prozessor konfiguriert ist, um:

den genannten Restgehalt mit dem Zielgehalt zu vergleichen und
die Behandlung (i) auszuwählen, wenn der Restgehalt kleiner oder gleich dem Zielgehalt ist,
andernfalls eine Datenbank oder ein Modell zu verwenden, das konfiguriert ist, um eine Schätzung des Gehalts an dem mindestens einem Heteroatom am Ende jeder Behandlung (ii) bis (iv) in Abhängigkeit vom Gehalt an diesem Heteroatom am Anfang der Behandlung, der gleich dem Restgehalt ist, bereitzustellen, und die Behandlung auszuwählen, bei welcher der geschätzte Gehalt am Ende der Behandlung kleiner oder gleich dem Zielgehalt ist und bei welcher optional die Anzahl der Behandlungsschritte am geringsten ist.

**15.** Bestimmungssystem nach Anspruch 14, wobei der mindestens eine Prozessor ferner konfiguriert ist, um:

(c) wenn keiner der geschätzten Gehalte am Ende kleiner oder gleich dem Zielgehalt ist, die Schritte (a) bis (c) zu wiederholen, nachdem die genannte Zusammensetzung natürlichen Ursprungs mit einer anderen Zusammensetzung natürlichen Ursprungs verdünnt wurde, optional mit einer Zusammensetzung, die einen für die Qualität der Zusammensetzung repräsentativen Parameter aufweist, der höher als derjenige der genannten Zusammensetzung ist, und/oder
(c) eine Behandlung und die Betriebsbedingungen dieser Behandlung auszuwählen, bei welcher der geschätzte Endgehalt kleiner oder gleich dem Zielgehalt ist, und optional bei welcher die Anzahl von Behandlungsschritten am geringsten ist und/oder bei welcher die Menge des verwendeten Reagenz am geringsten ist, wobei die Datenbank oder das Modell konfiguriert ist, um für jede Behandlung (ii) bis (iv) eine für verschiedene Betriebsbedingungen der Behandlungen (i) bis (iv) erhaltene Schätzung der Gehalte an dem mindestens einen Heteroatom am Ende der genannten Behandlung bereitzustellen.

## Claims

1. A method for determining a parameter representative of the quality of a composition of natural origin comprising heteroatoms and lipids selected from phenolic lipids, fatty acids, triglycerides, diglycerides, monoglycerides, phospholipids, fatty-acid esters and/or a mixture of two or more of these compounds, where the method comprises:

   (A) determining the total concentration of at least one heteroatom selected from phosphorus and nitrogen in said composition,
   (B) identifying the compounds with at least one heteroatom hydratable at neutral pH and determining the total concentration of said composition with at least one heteroatom present in the identified compounds with this heteroatom hydratable at neutral pH,
   (C) determining as parameter representative of the quality of the composition from the ratio of the total concentration of at least one heteroatom present in the identified compounds with this heteroatom hydratable at neutral pH to the total concentration of the composition with this heteroatom, where this ratio is determined for phosphorus, nitrogen or for each of these two heteroatoms.

2. The determination method according to claim 1, wherein step (B) is done by means of an analysis technique selected from NMR, mass spectroscopy, ion chromatography, liquid-phase chromatography coupled with mass spectrometry, and high-performance thin layer chromatography.

3. The determination method according to claim 1 or 2, wherein said composition of natural origin contains one or more oils selected from a vegetable oil, an animal oil or fat, a used oil, an oil produced by microorganisms, esters resulting from the transesterification of fatty-acid esters contained in one or more of these oils, and also mixtures thereof.

4. A process of determining the heteroatom elimination treatment to be applied to a composition of natural origin comprising heteroatoms and lipids selected from phenolic lipids, fatty acids, triglycerides, diglycerides, monoglycerides, phospholipids, fatty-acid esters and/or a mixture of two or more of these compounds for reaching a target concentration for at least one heteroatom, where the process comprises:

   (a) a step of determining the at least one parameter representative of the quality of the composition by means of the determination method, according to any one of claims 1 to 3,
   (b) a step of estimating a residual concentration of at least one heteroatom selected from phosphorus and nitrogen, based on the representative parameter, where this residual concentration corresponds to the concentration of at least one heteroatom after eliminating compounds hydratable at pH = 7 containing this heteroatom,
   (c) a step of determining a treatment to be applied to said composition for reaching a target concentration of the at least one heteroatom on output from the treatment, where said treatment is selected from:

      (i) washing with neutral pH water,
      (ii) washing with neutral pH water followed by a step of eliminating non-hydratable compounds,
      (iii) washing with neutral pH water followed by a step of eliminating compounds hydratable at a pH other than a neutral pH,
      (iv) washing with neutral pH water followed by a step of eliminating compounds hydratable at a pH other than a neutral pH then a step of eliminating non-hydratable compounds,

   wherein

   said residual concentration is compared to the target concentration, and
   the treatment (i) is selected if the residual concentration is less than or equal to the target concentration,
   otherwise, a database or a model is used configured for providing an estimate for a concentration of the at least one heteroatom on output from each treatment (ii) to (iv) as a function of a concentration of this heteroatom on input to the treatment equal to the residual concentration, and the treatment is selected for which the estimated output concentration is less than or equal to the target concentration, and optionally for which the number of treatment steps is fewer.

5. The determination process according to claim 4, wherein, during the step (c), if none of the estimated output concentrations is less than or equal to the target concentration, the steps (a) to (c) are iterated by diluting said composition of natural origin with another composition of natural origin, optionally a composition having a parameter

representative of the quality of the composition greater than that of said composition.

6. The determination process according to claim 4 or 5, wherein, the database or the model is configured for providing an estimate, for each treatment (ii) to (iv), of the concentrations of the at least one heteroatom on output from said treatment, obtained for various operating conditions of the treatments (i) to (iv) and during the step (c), a treatment and the operating conditions for this treatment are selected for which the estimated output concentration is less than or equal to the target concentration, and optionally for which the number of treatment steps is the fewest and/or for which the quantity of reagent used is the smallest.

7. The determination process according to any one of claims 4 to 6, wherein said composition of natural origin contains one or more oils selected from a vegetable oil, an animal oil or fat, a used oil, an oil produced by microorganisms, esters resulting from the transesterification of fatty-acid esters contained in one or more of these oils, and also mixtures thereof.

8. A process for reducing the heteroatom concentration of a composition of natural origin comprising heteroatoms and lipids selected from phenolic lipids, fatty acids, triglycerides, diglycerides, monoglycerides, phospholipids, fatty-acid esters and/or a mixture of two or more of these compounds, where the process comprises:

(E1) a step of determining the heteroatom elimination treatment to be applied to the composition of natural origin to reach a target concentration in at least one heteroatom, by means of the determination process according to any one of claims 4 to 7, and
(E2) a step during which said composition of natural origin undergoes the treatment determined in step (E1).

9. A process for reducing the heteroatom concentration of a composition of natural origin comprising heteroatoms and lipids selected from phenolic lipids, fatty acids, triglycerides, diglycerides, monoglycerides, phospholipids, fatty-acid esters and/or a mixture of two or more of these compounds, where the process comprises:
(E'2) a heteroatom elimination step during which the composition of natural origin undergoes a treatment selected from:

(i) washing with neutral pH water,
(ii) washing with neutral pH water followed by a step of eliminating non-hydratable compounds,
(iii) washing with neutral pH water followed by a step of eliminating compounds hydratable at a pH other than a neutral pH,
(iv) washing with neutral pH water followed by a step of eliminating compounds hydratable at a pH other than a neutral pH then a step of eliminating non-hydratable compounds, and wherein, the composition of natural origin has a parameter representative of the quality of the composition, previously determined by means of the determination method according to any one of claims 1 to 3, which is greater than or equal to a threshold beyond which the elimination step is sufficiently effective for reducing the heteroatom concentration of said composition to a target concentration or below the target concentration.

10. The reduction process according to claims 8 or 9, further comprising (E3) a step of hydrotreatment of the product coming directly from step (E2) or (E'2).

11. The reduction process according to any one of claims 8 to 10, wherein the composition of natural origin contains one or more oils selected from a vegetable oil, an animal oil or fat, a used oil, an oil produced by microorganisms, esters resulting from the transesterification of fatty-acid esters contained in one or more of these oils, and also mixtures thereof.

12. The reduction process according to any one of claims 8 to 11, wherein, during step (E2) or (E'2), the washing with neutral pH water for each of the treatments (i) to (iv) is done with the ratio by mass of water to composition from 1:99 to 10:90.

13. The reduction process according to any one of claims 8 to 12, wherein, during step (E2) or (E'2), the washing with neutral pH water for each of the treatments (i) to (iv) is done in an enclosure selected from a storage tank, a wash container, and a round bottom flask.

14. A system for determining the heteroatom elimination treatment to be applied to a composition of natural origin for reaching a target concentration in at least one heteroatom, configured for using steps (a) to (c) of the determination

process according to any one of claims 4 to 6, where the system comprises at least one processor configured for:

(a) receiving the total concentration in said composition of at least one heteroatom selected from phosphorus and nitrogen and the total concentration in said composition of hydratable compounds with at least one heteroatom, and for determining as a parameter representative of the quality of the composition, the ratio of the total concentration of at least one heteroatom present in the identified compounds with this heteroatom hydratable at neutral pH to the total concentration in the composition with this heteroatom, where this ratio is determined for phosphorus, nitrogen or each of these two heteroatoms,
(b) estimating a residual concentration of at least one heteroatom selected from phosphorus and nitrogen, based on the representative parameter, where this residual concentration corresponds to the concentration of at least one heteroatom after eliminating compounds hydratable at pH = 7 containing this heteroatom,
(c) determining a treatment to be applied to said composition for reaching a target concentration of the at least one heteroatom on output from the treatment, where this treatment is selected from:

(i) washing with neutral pH water,
(ii) washing with neutral pH water followed by a step of eliminating non-hydratable compounds,
(iii) washing with neutral pH water followed by a step of eliminating compounds hydratable at a pH other than a neutral pH,
(iv) washing with neutral pH water followed by a step of eliminating compounds hydratable at a pH other than a neutral pH then a step of eliminating non-hydratable compounds, where the at least one processor is configured for:

comparing said residual concentration to the target concentration, and
selecting the treatment (i) if the residual concentration is less than or equal to the target concentration, otherwise, using a database or a model configured for providing an estimate for a concentration of the at least one heteroatom on output from each treatment (ii) to (iv) as a function of a concentration of this heteroatom on input to the treatment equal to the residual concentration, and selecting the treatment for which the estimated output concentration is less than or equal to the target concentration, and optionally for which the number of treatment steps is fewer.

**15.** The determining system according to claim 14, wherein the at least one processor is further configured for:

(c) if none of the estimated output concentrations is less than or equal to the target concentration, iterating the steps (a) to (c) after dilution of said composition of natural origin with another composition of natural origin, optionally a composition having a parameter representative of the quality of the composition greater than that of said composition, and/or
(c) selecting a treatment and the operating conditions for this treatment for which the estimated output concentration is less than or equal to the target concentration, and optionally for which the number of treatment steps is fewer and/or for which the quantity of reagent used is less, where the database or the model is configured for providing an estimate for each treatment (ii) to (iv) of the concentrations of the at least one heteroatom on output from said treatment, obtained for various operating conditions of the treatments (i) to (iv).

[Fig. 1]

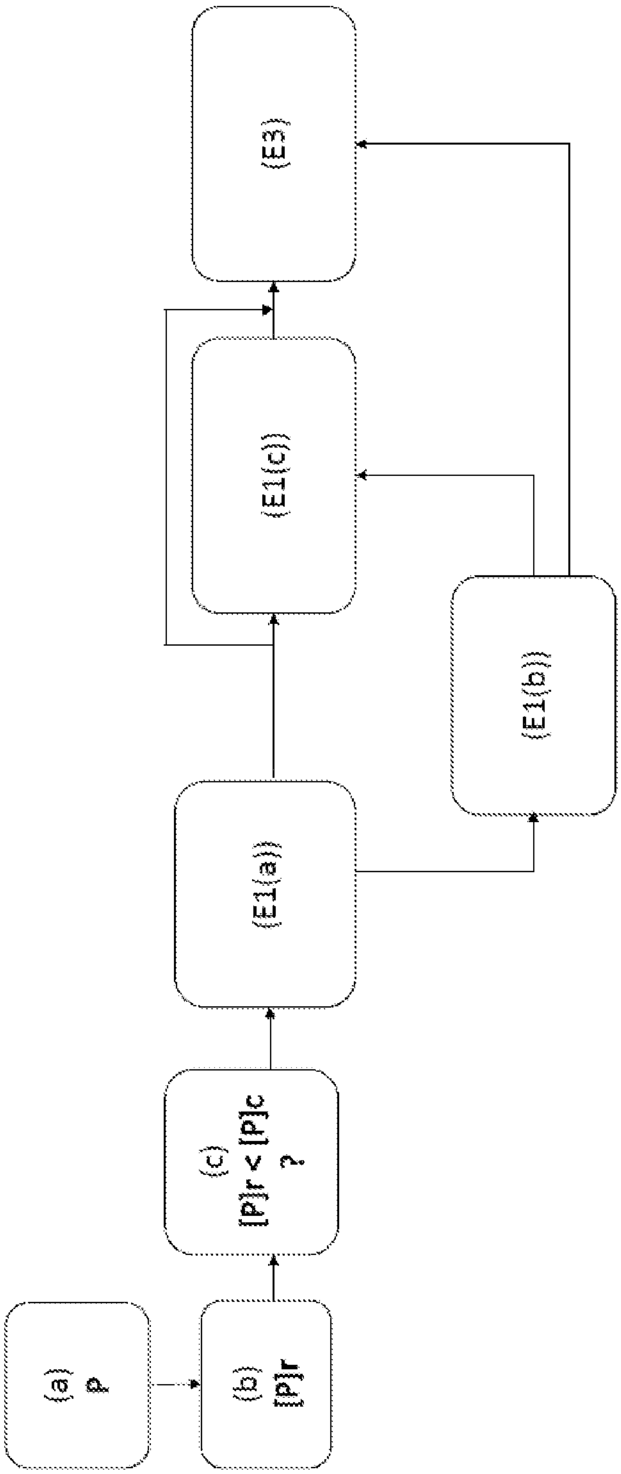


Figure 1

[Fig. 2]

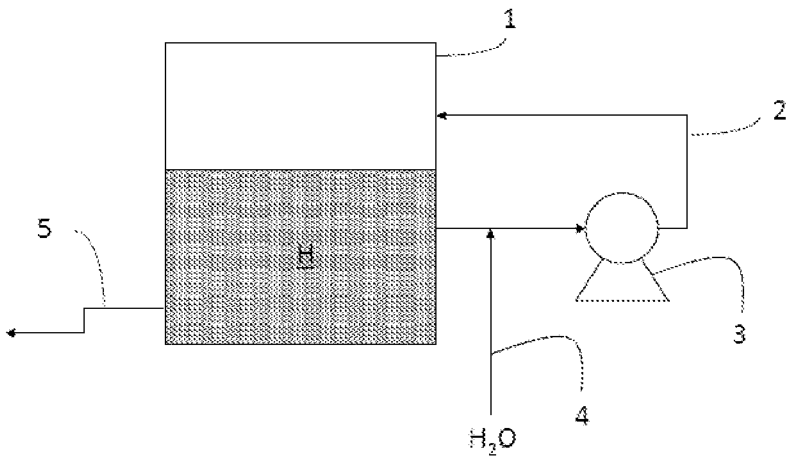


Figure 2

[Fig. 3]

<table>
<tr><th></th><th>PC</th><th>PE</th><th>PI</th><th>PA</th></tr>
<tr><td>pH <2-3</td><td rowspan="2">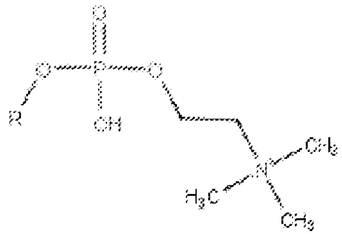</td><td>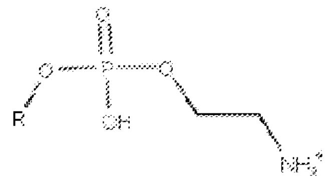</td><td rowspan="2">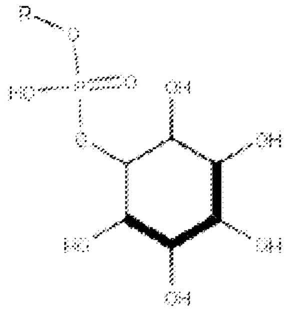</td><td rowspan="2">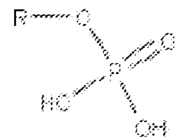</td></tr>
<tr><td>3<pH<4</td><td rowspan="2">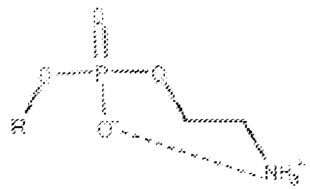</td></tr>
<tr><td>4<pH<7-8</td><td rowspan="2">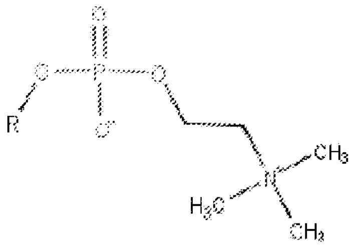</td><td rowspan="2">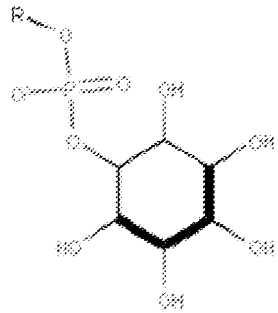</td><td>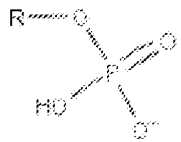</td></tr>
<tr><td>pH>8-9</td><td>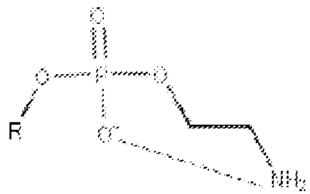</td><td>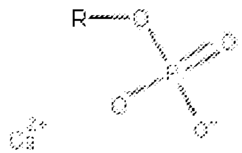</td></tr>
</table>

Figure 3

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 2019338219 A1
- US 2019031964 A1
- WO 201098783 A1
- US 8911808 B2
- US 7762715 B2
- US 8042989 B2
- WO 2019229035 A1


### Littérature non-brevet citée dans la description


- **VICTOR GARCIA-MONTOTO et al.** Phosphorus speciation analysis of fatty-acid-based feedstocks and fast pyrolysis biocrudes via gel permeation chromatography inductively coupled plasma high-resolution mass spectrometry. *RSC ADVANCES*, 05 August 2021, vol. 11 (43), 26732-26738
- The lipid handbook
- Lipids handbook
- **MONOJ. K. GUPTA**. practical guide to vegetable oil processing
- **T. GLONEK** ; **M. LUNDE** ; **M. MUDGETT** ; **T. C. MYERS**. Studies of Biological Polyphosphate Through the Use of Phosphorus-31 Nuclear Magnetic Resonance. *Archives of Biochemistry and Biophysics*, 1971, vol. 142, 508-513
- **T. O. HENDERSEN** ; **T. GLONEK** ; **T. C. MYERS**. Phosphorus-31 Nuclear Magnetic Resonance Spectroscopy of Phospholipids. *Biochemistry*, 1974, vol. 13 (3), 623-628